# EUROPÄISCHE PATENTSCHRIFT

(11) **EP 3 318 290 B1**
(45) Veröffentlichungstag und Bekanntmachung des Hinweises auf die Patenterteilung: **22.04.2020**
(21) Anmeldenummer: 16197018.1
(22) Anmeldetag: 03.11.2016
(51) Int. Cl.: A61M 1/00, A61F 9/007, G01L 19/00

(54) **WECHSELKASSETTE FÜR OPHTHALMOLOGISCHE APPARATUR**
CASSETTE FOR OPHTHALMOLOGICAL APPARATUS
CASSETTE POUR APPAREIL OPHTALMOLOGIQUE

(43) Veröffentlichungstag der Anmeldung: 09.05.2018
(73) Patentinhaber: This AG, 9435 Heerbrugg (CH)
(72) Erfinder: Köppel, Thomas, 9443 Widnau (CH); Zünd, Marco, 9443 Widnau (CH); Heiss, Martin Christoph, 9050 Appenzell Eggerstanden (CH)
(74) Vertreter: Kaminski Harmann

(56) Entgegenhaltungen:
- DE-A1-102009 037 845
- US-A- 5 364 342
- US-A- 5 746 719
- US-A1- 2002 107 468
- US-A1- 2003 190 244

## Beschreibung

Die Erfindung betrifft ein Wechselteil für eine ophthalmologische Apparatur nach dem Oberbegriff des Anspruchs 1 sowie ein zugehöriges Gerät nach Anspruch 10, als auch ein Verfahren zur Druckmessung, zur Herstellung einer Kopplung der Druckmessung und zur Herstellung des Wechselteils.

Die Erfindung betrifft augenchirurgische Apparaturen. Bei Engriffen innerhalb des Auges, erfolgt dabei in vielen Fällen eine Aspiration, also Absaugung von Augenflüssigkeit respektive von bei der Operation abgetragenen Partikeln. Zum Beispiel wird im Rahmen einer Kataraktoperation die getrübte Linse aus dem Auge entfernt und durch eine Intra-okularlinse(IOL)ersetzt.

Bei einer häufig angewandten Kataraktoperation wird meist eine Phakoemulsifikation (Linsenkernverflüssigung) durchgeführt, bei welcher beispielsweise über einen wenige Millimeter langen Schnitt im Bereich des Übergangs von Hornhaut zu Lederhaut Operationswerkzeuge ins Innere des Auges eingeführt werden. Es wird ein Stück der Kapsel entfernt und im Anschluss der Linseninhalt, z.B. mit einem Ultraschallgerät oder Laser, zerkleinert und entfernt. Das Entfernen der Linsenpartikel erfolgt dabei über eine an das Operationswerkzeug angeschlossenes Saugspülung, welches ein Kernpunkt der vorliegenden Erfindung darstellt. Diese Saugspülung, welche meist als Teil eines Operationsgeräts mit weiteren, für die Operation erforderlichen Funktionen ausgebildet ist, aspiriert die zertrümmerten Linsenkern-Teilchen und ersetzt das Abgesaugte durch Flüssigkeitsinfusion, beispielsweise durch eine Balanced Salt Solution (BBS). Nach dem Entfernen des alten Linsenkerns wird durch die Öffnung eine zusammengefaltete, elastische Ersatzlinse in den Kapselsack eingebracht.

Neben der oben beschriebenen Phakoemulsifikation kommen dieselben medizinische Geräte mit Saugspülfunktion, welche diese Erfindung behandelt - bzw. allenfalls spezifische Ausführungsformen derartiger Geräte - auch bei anderen ophthalmologischen Eingriffen zum Einsatz, bei welchen eine interokulare Aspiration oder eine Infusion oder beides durchgeführt wird, beispielsweise bei Vitrektomie, Diathermie, Kapsulotomie, Glaukom-Chirurgie, Refraktive Chirurgie, Femto-Laser, etc.

Beispielhaft beschreiben die Dokumente DE 696 21 562, US 2016/045367, WO 02/056850 exemplarische Ausführungen derartiger medizinischer Gerätschaften.

Für die Saugspülfunktion, also für die Infusion und/oder Aspiration wird ein entsprechendes medizinisches Gerät bereitgestellt, welches die Zu- und Abfuhr der Flüssigkeiten, sowie allfällige weitere Funktionen wie etwa die Ansteuerung der Phakoemulsifikation bereitgestellt. Dazu verfügt das Chirurgische Gerät, mit welchem in das Auge eingegriffen wird, über eine Aspirationsleitung zur Absaugung und eine Infusionsleitung zum Ersatz der abgesaugten und/oder durch die Einführungsöffnung austretenden Flüssigkeit. Speziell sollte dabei auch während der Operation der Augendruck (IOP = Inter Okular Pressure) zumindest annähernd konstant gehalten werden, sodass das Auge, bzw. ein Augenteil wie die Vorderkammer, nicht zusammenfällt oder aufgebläht wird, weshalb parallel zur Absaugung durch die Aspiration gleichzeitig eine Infusion, also Zuführung von Flüssigkeit erfolgt.

Um Sterilität zu gewährleisten, muss - insbesondere bezüglich der aspirierten Flüssigkeit, welche potentiell kontaminiert ist - eine intraokulare Infektion oder Übertragung von Patient zu Patient sicher vermieden werden. Dies kann unter anderem beispielsweise durch Austausch eines Wechseleinsatzes, welcher meist als Kassette bezeichnet wird, erzielt werden. Diese Kassetten können als Einwegkassetten, Tageskassetten und/oder auch mehrfach sterilisierbar ausgeführt sein. Der Kassette kommt dabei insbesondere die Bereitstellung der Aspirationswirkung zu, und enthält insbesondere jene Teile, welche mit potentiell kontaminierter Patientenflüssigkeit in Berührung gelangen oder gelangen könnten. Speziell können diese Teile etwa Pump- bzw. Absaugvorrichtungen, Ventile und/oder Sensoren für Druck und Unterdruck, Luftblasen, Durchfluss, etc. sein. Auch - vorzugsweise gegen Vertauschung mechanisch codierte - Anschlüsse zu Leitungen von und/oder zum Patienten, sowie für Anschlüsse für Abfall- und/oder Infusionsbehälter, etc. sind meist Teil einer derartigen austauschbaren Kassette, welche in austauschbarer Weise zumindest teilweise an oder im medizinischen Gerät angebracht wird.

Die Dokumente DE 693 09 757, DE 693 21 264, DE 696 15 507, DE 696 15 633, DE 697 09 192, US 5,163,900, US 5,282,787, US 5,582,601, US 5,800,396, US 5,897,524, US 5,899,674, WO 1987/001943, WO 2004/108189, oder WO 2004/110524 zeigen artverwandte Kassetten. Einwegelemente kommen auch in artfremden Gebieten, wie z.B. in Analysegeräten wie in DE 10 2009 037845 oder im Dialysegerät aus US 2002/107468 vor, wenn auch mit gattungsmäßig gänzlich anderen technischen Anforderungen und entsprechenden Strukturen. Um die Aspirationswirkung zu erzielen, werden unterschiedliche Pumpsysteme für die aspirierte Flüssigkeit eingesetzt. Insbesondere Systeme mit Peristaltikpumpen oder Systeme mit einer Absaugung durch einen, von einer Venturi-Düse erzeugten, Luft-Unterdruck sind dabei konkurrierende Systeme, wobei aufgrund Ihrer unterschiedlichen Aspirationscharakteristiken bei verschiedenen Anwendungsfällen oder aufgrund von persönlichen Präferenzen verschiedenen Chirurgen das eine oder das andere System bevorzugt wird. Es können beispielsweise aber auch andere Flüssigkeitsfördersystem wie z.B. Membranpumpen, etc. zur Aspiration genutzt werden. WO 2010/129128 zeigt ein Beispiel einer Peristaltik-Pumpe in einer Kassette.

Vorzugsweise werden Sensorik, Aktoren, Elektronik und/oder Mechanik möglichst im medizinischen Gerät und nicht in der austauschbaren Kassette untergebracht. Die Kassette weist dabei also insbesondere nur die mit potentiell kontaminiertem Fluid in Kontakt tretender Teile der Sensorik und der Aktoren auf, also z.B. Messkammer, Membrane, Pump- oder Saugeinrichtungen, Quetschbereiche von Ventilen, etc. - also insbesondere passive Teile. Die aktiven Teile von Sensorik und Aktoren, insbesondere wenn diese einen beträchtlichen Kostenfaktor darstellen, werden vorzugsweise im medizinischen Gerät untergebracht. Auch im Sinne einer sinnvollen Widerverwertbarkeit und Recycling-Aspekten empfiehlt sich eine derartige Aufteilung. Beispielsweise kann die Kassette dabei als reines Kunststoffteil ausgebildet werden, welches vorzugsweise keine Elektronik- und/oder Metall-Komponenten enthält - was unter anderem eine sachgerechte Entsorgung erleichtert.

Drucksensoren, welche nur Überdruck zu detektieren haben, sind dabei verhältnismäßig einfach implementierbar. Beispielsweise kann eine Membrane im Flüssigkeitskreislauf der Kassette vorgesehen sein, welche derart ausgebildet ist, dass mit einer Kraftmessung an dieser eine Erfassung des Druckes der Flüssigkeit (gegenüber der Außenluft) durchführbar ist. Die Kraftbestimmung kann dabei z.B. in bekannter Weise, insbesondere mittels Dehnungsmessstreifen, piezo elektrischen Sensoren, magnetischen Sensoren, etc. erfolgen.

Alternativ sind auch Drucksensoren bekannt, bei welchen eine geometrische Auslenkung einer federnden Membrane (oder vergleichbarem) vom Flüssigkeitsdruck im Kreislauf abhängt, vorzugsweise in definierter und bekannter Abhängigkeit. Eine Membrane kann in dieser Alternative dann von einem Sensor in Ihrer geometrischen Lage ausgewertet werden. Beispielsweise erfolgt in US 5 364 342 eine Druckmessung durch eine kapazitive Messung einer Lage eines galvanisierten Elements in einer Druckmesskammer. Dabei kann alternativ zur Ausschlags-Methode auch eine Kompensations- oder Nullabgleichs-Methode bei der Messung angewandt werden. Im Allgemeinen kann aber mit einer Kraftmessung, insbesondere bei vernachlässigbar geringer geometrischer Auslenkung der Membrane, eine genauere und langzeitstabilere Druckerfassung erfolgen, weshalb vorzugsweise dieses Prinzip zum Einsatz kommt.

Bei einer Druckerfassungseinrichtung, welcher zur Erfassung von Über- als auch von Unterdruck ausgebildet ist, muss dabei die Kraft, respektive die Auslenkung in positiver als auch in negativer Richtung erfassbar sein. Ein Vorteil einer Kraftmessung besteht z.B. darin, dass durch die sehr geringe Auslenkung der Membran keine zusätzliche Elastizität in den Aspirationspfad gebracht wird. Dadurch wird die Druckstabilität im Auge, insbesondere beim Lösen einer Okklusion bedeutend, erhöht. Entsprechend muss daher eine Anbindung der Druckmessmembrane der Kassette an die zugehörige Druckmess-Sensorik im Gerät zur Übertragung von positiven als auch negativen Werten ausgebildet sein, oder alternativ ein sogenanntes Biasing - also ein Verschieben des Arbeitspunktes (beispielsweise durch einen konstanten Offset) erfolgen. Die mechanische Ausbildung wird daher entsprechend komplexer. Beispiele solcher bekannten Druckerfassungseinrichtungen finden sich etwa in EP 1 253 412, US 5,392,653 oder US 3,841,157.

Beispielsweise zeigt auch das Dokument DE 600 00 608 ein Druckmesssystem, bei welchem eine über eine Kammer gestülpte Membran einen Ring aufweist, in welchem eine Platte aufgenommen wird. Mittels dieser Platte wird ein Kraftaufnehmer entfernbar mit der Membran gekoppelt. Das Koppeln kann dabei, ähnlich wie in DE 693 16 209, vorzugsweise durch eine ferromagnetische Platte und einen Magnet am Kraftaufnehmer erfolgen. Alternativ wird die Verwendung einer Rasteinrichtung vorgeschlagen, welche als eine Befestigung an der Platte ausgebildet ist und mit einer Buchse am Kraftaufnehmer mittels einer Klemmeinrichtung (wie etwa einer Madenschraube) lösbar gekoppelt werden kann. Unter anderem nachteilig ist hierbei, dass die Kassette zur magnetischen Kopplung ein Metallteil aufweisen muss. Auch das Koppeln und Lösen der magnetischen Verbindung mit Permanentmagneten stellt eine hohe mechanische Belastungen für die Membrane dar, aber auch die Alternative mit einem abschaltbaren Elektromagneten ist nicht frei von diesen Nachteilen und erfordert zudem eine dauerhafte Bestromung während der ganzen Betriebsdauer. Auch ist in beiden Fällen keine exakte Reproduzierbarkeit der Kopplung gegeben, sodass bei jedem Einlegen unterschiedliche Messcharakteristiken auftreten können. Auch der vorgeschlagene Einsatz einer Madenschraube ist im praktischen OP-Umfeld wenig praktikabel. Zudem ist das Bauvolumen der darin gezeigten Lösung eher groß, und auch die Richtung (orthogonal zur Messmembrane), in welcher das Koppeln bei der gezeigten Lösung erfolgen muss, ist bezüglich einer Implementierung in eine ophthalmologische Kassette und für deren Einlegevorgang unvorteilhaft.

Es ist eine Aufgabe der vorliegenden Erfindung, eine ophthalmologische Kassette zu verbessern bzw. eine verbesserte Kassette bereitzustellen.

Eine Aufgabe ist dabei, auch die Druckmesseinheit zu verbessern und zu vereinfachen, und eine ebensolche in der Kassette bereitzustellen.

Es ist insbesondere eine Aufgabe dabei eine einfache, sichere und zuverlässige Kopplung zwischen Kassette und Gerät bereitzustellen, welche auch viele Wechselzyklen übersteht.

Die Verbesserung und Vereinfachung der Kassette bezüglich ihrer Zusammenwirkung mit dem Gerät, insbesondere beim Einlegen und Entfernens während des Wechselvorgangs, ist eine weitere Aufgabe der vorliegenden Erfindung.

Als spezielle Aufgabe ist dabei eine Kassette mit kompaktem Aufbau bereitzustellen und/oder die Recyclingfähigkeit zu verbessern.

Es ist auch eine spezielle Aufgabe, mögliche Fehlerquellen bei der Handhabung sowie bei der eigentlichen Verwendung der ophthalmologischen Kassette zu reduzieren.

Es ist auch eine Aufgabe eine funktional flexible und vielseitige Kassette bereitzustellen, beziehungsweise ein einziges Gerät zur flexiblen Nutzung von unterschiedlichen Kassetten-Optionen mit jeweils spezifischen, dem Anwendungsfall angepassten Funktionalitäten bereitzustellen. Dabei sollten die entsprechenden Kassetten-Optionen vorzugsweise zueinander Kompatibel sein.

Diese Aufgaben werden erfindungsgemäß durch die Merkmale der unabhängigen Ansprüche und/oder durch Merkmale der abhängigen Ansprüche gelöst bzw. diese Lösungen weitergebildet.

Die vorliegende Erfindung betrifft ein Wechselteil für eine ophthalmologische Apparatur. Dabei kann das Wechselteil speziell als eine, mit Patientenflüssigkeit in Kontakt tretende und austauschbare ophthalmologische Kassette für ein ophthalmo-chirurgisches Gerät ausgebildet sein. Beispielsweise etwa ein Gerät zur Phakoemulsifikation, Vitrektomie, Diathermie, Femto-Lasern, Capsulotomie, etc.

Das Wechselteil ist dabei aufgebaut mit zumindest einem Hartbereich als Gehäuse - insbesondere aus einem Hartkunststoff, speziell einem Thermoplast wie z.B. einem Polypropylen (PP) oder Polyethylen (PE) - und zumindest einem Weichbereich - insbesondere aus einem Weichkunststoff wie z.B: einem thermoplastischen Elastomer (TPE), einem Elastomer, oder einem Silikon. Dabei bilden der Hartbereich und der Weichbereich interne Flüssigkeitskanäle des Wechselteils aus. Der Weichbereich bildet dabei auch Funktionselemente des Wechselteils aus, zumindest
▪ eine Pumpeinrichtung für Flüssigkeit in den Flüssigkeitskanälen, insbesondere in Form einer Peristaltikpumpe,
▪ ein Ventileinrichtung zur Veränderung eines Durchflussquerschnitts in zumindest einem der Flüssigkeitskanäle, und
▪ eine Druckerfassungseinrichtung zur Bestimmung eines Flüssigkeitsdruckes in zumindest einem der Flüssigkeitskanäle.

Das Wechselteil hat weiters einen Aspirationsanschluss zur Verbindung von zumindest einem der Flüssigkeitskanäle mit einem ophthalmo-chirurgischen Operationsinstrument, und einem Abfall-Anschluss zur Abfuhr von, insbesondere aspirierter, Flüssigkeit in ein Abfallgebinde.

Die Drucksensoreinrichtung ist dabei ausgebildet mit einer Druckkammer, welche mit einem der Flüssigkeitskanäle in Verbindung steht, und einer flexiblem Membrane welche ein Kupplungselement umlaufend einfasst. Dabei kann die Membrane insbesondere als Weichbereich ausgebildet sein, und insbesondere zumindest annähernd kreisrund sein und mit dem Kupplungselement zumindest eine gemeinsame Mittelachse aufweisen.

Das Kupplungselement ist dabei mit einer, insbesondere zumindest teilweise umlaufenden, Ausnehmung derart ausgebildet, dass ein gabelförmiger Ausleger einer Kraftmesseinrichtung einschiebbar ist. Dieser Ausleger ist dabei substantiell parallel zur Membrane ausgerichtet und derart direkt in die Ausnehmung einschiebbar, dass das Kupplungselement zumindest zweiseitig gefasst ist. Vorzugsweise umfasst die Ausnehmung des Auslegers das Kupplungselement über einen Winkelbereich von mehr als 180°. Dabei ist mit dem Ausleger eine, in substanziell normaler Richtung zur Membrane auftretende Kraftwirkung auf die Membrane erfassbar. Es erfolgt dabei eine direkte Kopplung des kraftmessenden Auslegers, welcher insbesondere als eine mit Dehnmessstreifen versehene Platte mit gabelförmiger Ausnehmung zum direkten Eingriff in das Kupplungselement an einer Seite und einer festen Verbindung zum Gerät an der anderen Seite. Vorzugsweise sind dabei die Dehnmessstreifen an einer Stelle des Auslegers angebracht an welcher seine Querschnittsfläche verringert ist.

Mit anderen Worten, hat ein erfindungsgemäßes Wechselteil eine Druckmesskammer, welche an einer, insbesondere substantiell plan ausgeformten, Außenseite ein Kupplungselement aufweist, welches umlaufenden von einer flexiblen Membrane eingefasst ist. Das Kupplungselement ist dabei ausgebildet mit einer - insbesondere zumindest teilweise umlaufenden - Ausnehmung, welche derart ausgebildet ist, dass in diese Ausnehmung, vorzugsweise formschlüssig von zumindest zwei Seiten eine gabelförmiger Ausleger einer Kraftmesseinrichtung eingreifbar ist, wobei der Ausleger zumindest annähernd parallel zur Membrane ausgerichtet ist. Dabei bewirkt der auf die Membrane wirkender Druck der sich in der Druckkammer befindlichen Flüssigkeit, eine substantiell in Normalrichtung zur Membrane wirkende Kraftwirkung, welche über das Kupplungselement auf den, um das Kupplungselement eingreifenden gabelförmigen Ausleger der Kraftmesseinrichtung übertragen wird. Entsprechend kann der Druck sowohl als Unter- als auch als Überdruck gegenüber dem umgebenden Atmosphärendruck bestimmt werden, da die Kraftwirkung in positiver sowie in negativer Richtung übertragbar ist.

Die Druckkammer kann dabei erfindungsgemäß als Hartbereich (bzw. als Bereich des Hartbereichs) und die Membrane und das Kupplungselement als Weichbereich (bzw. als Teilbereich des Weichbereichs) ausgebildet sind.

Die Membrane und das Kupplungselement können dabei einstückig ausgebildet sind, speziell einstückig als Teil des Weichbereichs.

Der Weichbereich (oder ein Teilbereich des Weichbereichs) kann auch - insbesondere interne - Dichtungen der Druckmesskammer und der Flüssigkeitskanäle des Wechselteils ausbilden.

Zur Steuerung, Regelung und/oder Überwachung der Vorgänge während einer Operation sind neben den Aktoren, welche Flüssigkeitsbewegungen aktiv beeinflussen, auch Sensoren im Flüssigkeitskreislauf erforderlich. Insbesondere werden dabei Druckverhältnisse im Flüssigkeitskreislauf ermittelt. Speziell da Luftblasen in einem derartigen hydraulischen System die Druckverhältnisse verfälschen könnten und/oder ein Einbringen von Luft in das Auge vermieden werden muss, kann vorzugsweise auch zumindest eine Überwachung auf Luftblasen im ophthalmologischen System implementiert sein.

Das Wechselteil kann zudem einen Flaschenanschluss zum Anschluss eines Behälters zur Bereitstellung von Infusionsflüssigkeit, und einen Infusionsanschluss zur Zuführung der Infusionsflüssigkeit zum ophthalmo-chirurgischen Operationsinstrument, aufweisen, welche intern über separate Flüssigkeitskanäle verbunden sind. Dabei können die separaten Flüssigkeitskanäle insbesondere ein Infusions-Ventil zur Steuerung eines Infusionsdurchflusses aufweisen. Alternativ oder zusätzlich können die separaten Flüssigkeitskanäle auch eine Infusions-Pumpeinrichtung und/oder einen Infusions-Drucksensor aufweisen.

Der Hartbereich kann dabei aus einem Hartkunststoff und der Weichbereich aus einem thermoplastischen Elastomer ausgebildet sein, welche - insbesondere zumindest für einzelne Teile des Wechselteils - als gemeinsames, einstückiges Mehrkomponenten-Spritzgussteil ausgebildet sein können.

Der Hartbereich kann dabei Aussparungen aufweisen, durch welche Bereiche des Weichbereichs, welche vorzugsweise die Funktionselemente ausbilden, vom Äußeren des Wechselteils mechanisch zugänglich sind. Insbesondere können diese Bereiche zumindest Teile der Funktionsbereiche von der Drucksensoreinrichtung, Druckerfassungseinrichtung, von den Flüssigkeitskanälen der Pumpeinrichtung, speziell von Quetschbereichen einer Peristaltikpumpe, von Ventileinrichtungen und/oder von aktiven oder passiven Ripple-Kompensationseinrichtungen ausbilden.

Das Wechselteil kann in erfindungsgemäßen Ausführungsformen auch zumindest einen Luftblasendetektor zur Detektierung von Luft in den Flüssigkeitskanälen aufweisen, welche insbesondere optisch, speziell via Refraktionseffekten arbeiten. Beispielsweise kann der Hartbereich dazu aus optisch transparentem Material ausgebildet sein, welches für den Luftblasendetektor ein, insbesondere poliertes, Sichtfenster ausbildet. Speziell kann dabei die Auswerteelektronik des Luftblasendetektors außerhalb des Wechselteils im Gerät fest verbaut sein.

Das Wechselteil kann in erfindungsgemäßen Ausführungsformen auch mit zumindest einer Ventileinrichtung zur Steuerung des Durchflusses in einem der Flüssigkeitskanäle ausgebildet sein. Speziell kann dabei die Ansteuerungselektronik und Mechanik des Ventils außerhalb des Wechselteils im Gerät fest verbaut sein und lediglich auf einen Weichbereich des Wechselteils mechanisch einwirken.

Das Wechselteil kann in erfindungsgemäßen Ausführungsformen auch mit zumindest einer Ripple-Kompensation des Peristaltik-Effekts zwischen Fluid-Anschluss und Pumpeinrichtung ausbildet sein. Diese kann insbesondere aktiv mittels eines Aktuators oder passiv mittels eines elastischen Druckfluktuations-Ausgleichsbereichs ausgebildet sein.

In einem erfindungsgemäßen Wechselteil können insbesondere auch die Flüssigkeitskanäle und Funktionselemente derart ausgebildet und angeordnet sein, dass in Einbaulage die Flussrichtung der Flüssigkeit beispielsweise vom Aspirationsanschluss zum Abfallgebinde im Wechselteil substantiell entgegen der Gravitationsrichtung verläuft, sodass allfällige Luftblasen abgeführt werden.

Das Wechselteil kann auch eine äußere Verzahnung im Hartbereich aufweisen, welche dazu ausgebildet ist, dass das Wechselteil von der ophthalmologischen Apparatur automatisch einziehbar und im dieser positionierbar ist.

Die Erfindung betrifft ebenfalls eine entspreche ophthalmologische Apparatur, also ein Gerät für die Augenchirurgie, insbesondere mit welchem eine Saugspülung während der Operation durchführbar ist, welches zur Aufnahme einer Ausführungsform eines erfindungsgemäßen Wechselteils ausgebildet ist. Hat dabei insbesondere einen Aufnahmeschacht für das Wechselteil, welcher Aktoren und Sensoren aufweist, die ausgebildet sind um mit den Funktionselementen des Wechselteils interagieren können. Insbesondere weist das Gerät einen Ausleger einer Kraftmesseinrichtung auf, welcher mit einem Kupplungselement einer Druckmessmembrane einer erfindungsgemäßen Kassette bei linearem einschieben der Kassette eine Wirkverbindung eingeht. Diese Wirkverbindung ist dabei insbesondere beinahe spielfrei in orthogonaler Richtung zur Membrane und wird mittels eines gabelförmigen Endes des Auslegers, welches zumindest zweiseitig in das Kupplungselement eingreift beim Einschieben hergestellt.

Ebenso betrifft die Erfindung ein entsprechendes Kopplungsverfahren für ein Wechselteil mit einer ophthalmologische Apparatur, bei welchem insbesondere ein Koppeln eines Druckmesssensors erfolgt, bei welchem ein Einschieben einer gabelförmigen Ausbuchtung eines Auslegers einer Kraftmesseinrichtung in eine zumindest teilweise umlaufende Ausnehmung in einem, mit einer Membrane im Flüssigkeitskanal fest verbundenen, Kupplungselement. Vorzugsweise erfolgt das Einschieben linear in eine Einlegerichtung der Kassette in das Gerät.

Auch ein Teil der Erfindung ist ein entsprechendes Verfahren zur Aspirationsdruckmessung mit einer ophthalmologischen Kassette in einem ophthalmologischen Gerät, wobei mit einer kassettenseitigen Druckmessmembrane, welche einen Kupplungselement aufweist, in welches ein gabelförmiger Ausleger einer geräteseitigen Kraftmesseinrichtung zumindest zweiseitig umschließend eingreift, wobei die Druckmessmembrane und der Ausleger substantiell parallel zueinander ausgerichtet sind.

Dabei erfolgt ein Eingriff der Gabel von zumindest zwei Seiten in die Ausnehmung des Kupplungselements, wobei die Gabel dann in Normalrichtung zur Membrane über das Kupplungselement mit der Membrane beinahe spielfrei verbunden ist. Bei der Druckmessung erfolgt die Erfassung eines Werts der Kraftwirkung in Normalrichtung zur Membrane, wobei die geometrische Auslenkung der Membrane und der Gabel vernachlässigbar gering bleibt. Die beim Einlegen der Kassette lösbar hergestellte Verbindung von Kupplungselement und Gabel ist dabei - speziell in Normalrichtung zur Membranenfläche - vorzugsweise beinahe spielfrei, hat also beispielsweise ein Spiel in einer Größenordnung von einigen hundertstel oder zehntel Millimetern. Ein derartiges, geringes Spiel ermöglicht, dass das Koppeln sicher und mit geringem Kraftaufwand erfolgen kann indem die Zinken der Gabel in die seitlichen Ausnehmungen des Kupplungselements gleiten, und keine wesentlichen Verformungen von Kupplungselement und/oder Membrane auftreten und insbesondere nach dem Koppeln in paralleler Richtung zur Membrane nur vernachlässigbar geringe Kräfte auftreten. Ein komplett spielfreies Koppeln ist erfindungsgemäß auch nicht zwingend erforderlich, da nicht eine geometrische Auslenkung sondern eine Kraftwirkung bestimmt wird, auf welche ein allfälliges geometrisches Spiel nur vernachlässigbar geringe Auswirkungen hat. Entsprechend ist es auch nicht zwingend erforderlich, dass das Kupplungselement in Richtung der zu messenden Kraft rigide ausgeführt ist, sondern das Kupplungselement kann durchaus eine gewisse Flexibilität aufweisen da diese auf den zu bestimmende Kraftwert ebenfalls nur eine meist vernachlässigbar geringe Auswirkung hat. Die vom Druck der Flüssigkeit auf die Membran ausgeübte und via dem Kupplungselement auf den Ausleger übertragene Kraft ist dabei ein Maß für den Flüssigkeitsdruck, insbesondere eine positive Kraft für einen Überdruck gegenüber der Atmosphäre und eine negative Kraft für einen Unterdruck gegenüber der Atmosphäre.

Weiters ist ein vorteilhaftes Verfahren zur Herstellung einer erfindungsgemäßen ophthalmologischen Kassette, welche auf zumindest zwei gegenüberliegenden Seiten mechanisch flexible Funktionselemente aufweist. Das Verfahren beinhaltet ein Mehrkomponenten-Spritzgießen von einem, einen Hartbereich bildenden, Hartkunststoff und einem, zumindest ein Funktionselement bildenden, Weichkunststoff. Dabei erfolgt eine Ausbildung einer ersten als auch einer zweiten, gegenüberliegenden Seitenschale der Kassette. Bei der Herstellung erfolgt dann ein unlösbares Verschnappen oder Verpressen der ersten und zweiten gegenüberliegenden Seitenschale, wobei der Weichkunststoff Dichtungen von Flüssigkeitskanälen im Inneren der Kassette ausbildet. In einer weiteren Ausführungsform kann vor dem Verschnappen zwischen erster und zweiter Seitenschale ein Kernteil, insbesondere aus Hartkunststoff eingelegt werden.

Einige hier beschriebene Verfahren werden vorzugsweise zumindest teilweise als programmierte Abläufe bereitgestellt. Die Erfindung betrifft deshalb auch Computerprogrammprodukte mit Programmcode, der auf einem maschinenlesbaren Träger gespeichert ist, oder als ein Computer-Daten-Signal, verkörpert durch eine elektromagnetische Welle (z.B. etwa als Funksignal) bereitgestellt ist, zur Durchführung des obigen Verfahrens. Dabei kann der Programmcode speziell ein automatisches Durchführen von hier angeführten Steuerungs- und/oder Regelungs-Aufgaben, sowie von Ablaufsequenzen in einem ophthalmologischen Gerät ausführen bzw. hierzu ausgebildet sein.

Das erfindungsgemäße Verfahren und die erfindungsgemäße Vorrichtung werden nachfolgend anhand von in den Zeichnungen schematisch dargestellten, konkreten Ausführungsbeispielen rein beispielhaft näher beschrieben, wobei auch auf weitere Vorteile der Erfindung eingegangen wird. Im Einzelnen zeigen:
Fig. 1 eine erste Ausführungsform eines erfindungsgemäßen Wechselteils für ein ophthalmologisches Gerät als Block-Schema;
Fig. 2 eine zweite Ausführungsform eines erfindungsgemäßen Wechselteils für ein ophthalmologisches Gerät als Block-Schema;
Fig. 3a und Fig. 3b eine erste Ausführungsform eines Beispiels eines erfindungsgemäßen Wechselteils in Form einer austauschbaren Kassette für ein ophthalmologisches Gerät in 3D Ansichten von links und rechts;
Fig. 3c eine Schnittdarstellung der ersten Ausführungsform eines erfindungsgemäßen Wechselteils;
Fig. 3d eine Explosionsdarstellung der ersten Ausführungsform eines erfindungsgemäßen Wechselteils;
Fig. 4a, Fig. 4b und Fig. 4c eine Darstellung einer beispielhaften erfindungsgemäßen Ausführungsform eines Wechselteils, welches in ein entsprechendes Beispiel eines erfindungsgemäßen ophthalmologischen Geräts eingelegt ist;
Fig. 5, Fig. 6a, Fig. 6b, Fig. 6c, Fig. 6d , Fig. 6e und Fig. 6f Ausführungsformen von Beispielen eines erfindungsgemäßen Wechselteils mit Focus auf die Kopplung der erfindungsgemäßen Druckerfassungseinrichtung;
Fig. 7a, Fig. 7b, Fig. 7c und Fig. 7d Ausführungsformen eines Beispiels eines erfindungsgemäßen Wechselteils mit Focus auf die Kopplung der erfindungsgemäßen kassettenseitigen Druckerfassungseinrichtung;
Fig. 8a, Fig. 8b, Fig. 8c und Fig. 8d eine Darstellung einer beispielhaften erfindungsgemäßen Ausführungsform eines Wechselteils mit erfindungsgemäßer Druckerfassungseinrichtung und erfindungsgemäßer Pumpeinrichtung sowie deren Zusammenspiel;
Fig. 9a und Fig. 9b eine Ausführungsform eines Beispiels eines erfindungsgemäßen Wechselteils mit Focus auf ein erfindungsgemäßes kassettenseitiges Ventil;
Fig. 10 eine Ausführungsform eines Beispiels eines erfindungsgemäßen Wechselteils mit Focus auf einen erfindungsgemäßen kassettenseitigen Überdrucksensor;
Fig. 11 eine Ausführungsform eines Beispiels eines erfindungsgemäßen Wechselteils mit Focus auf einen erfindungsgemäßen kassettenseitigen Luftblasensensor.

Die Darstellungen in den Figuren dienen lediglich zur Illustration und sind, sofern nicht explizit angegeben, nicht als maßstäblich zu betrachten. Gleiche oder funktionell ähnliche Merkmale sind, insoweit praktikabel, durchgängig mit denselben Bezugszeichen versehen und werden gegebenenfalls mit einem Buchstaben als Index unterschieden. Die dargestellten Schemata zeigen jeweils die technische Grundstruktur, welche von einem Fachmann entsprechend allgemeinen Grundsätzen ergänzt oder modifiziert werden können.

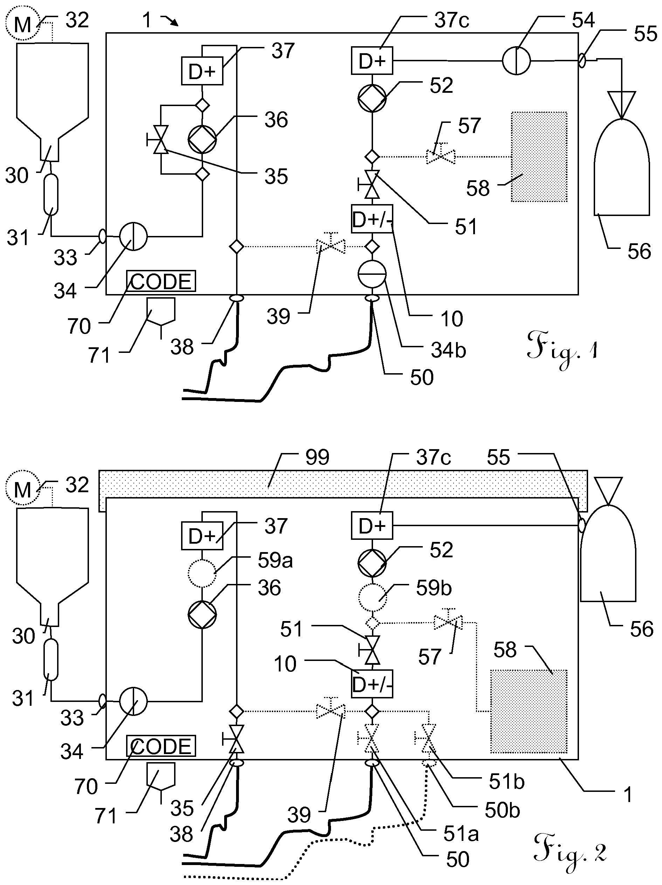

**Fig. 1** zeigt ein mögliches Beispiel einer Ausführungsform einer erfindungsgemäßen ophthalmologische Kassette 1 mit zumindest einer erfindungsgemäßen Druckerfassungseinrichtung 10 als Blockdiagramm. Es kann sich dabei speziell um eine Druckerfassungseinrichtung 10 in einer austauschbaren Kassette 1 eines ophthalmologischen Geräts 99, mit welchem eine Saugspülung durchführbar ist, handeln. In der linken Hälfte der Figur ist eine Infusionseinrichtung gezeigt, in der rechten Hälfte eine Aspirationseinrichtung. Ein Behälter 30, also beispielsweise eine Infusionsflasche oder ein Infusionsbeutel, stellt dabei eine Infusionsflüssigkeit bereit. Diese Infusionsflüssigkeitszufuhr kann z.B. mit einer Einrichtung zur Kontrolle des Füllstandes ausgestattet sein. Beispielsweise kann mit einem Sensor der Behälter 30 und/oder eine Tropfkammer 31 auf Entleerung überwacht werden, und zeitgerecht eine entsprechende Warnung ausgegeben werden. Wie im Stand der Technik bekannt, kann durch eine, vorzugsweise motorisiert und automatisch erfolgende, Höhenverstellung der Aufhängung des Behälters 30, welche hier durch den Antrieb 32 symbolisiert wird, der Infusionsflüssigkeitsdruck am Flaschen-Anschluss 33 der Kassette 1, (bzw. im gesamten Infusionssystem) durch Variation der Höhe der Behälteraufhängung verändert oder geregelt werden. Der Anschluss 33 kann dabei - in eingelegtem Zustand der Kassette 1 von außen zugänglich - an der Kassette 1 angeordnet sein, und beispielsweise über einen Schlauch mit dem Infusionsbehälter 30, respektive mit der dazwischenliegenden Tropfkammer, verbunden werden.

Die erfindungsgemäße Kassette 1 kann dabei zumindest einen Luftblasensensor 34 im Infusionssystem aufweisen, mit welchem eine korrekte Funktion überwacht und eine Infusion von Luft vermieden werden kann. Der Luftblasendetektor 34 kann dabei insbesondere optisch - beispielsweise aufgrund unterschiedlicher Lichtbrechungseigenschaften von Luft und Flüssigkeit, oder auch auf anderen Prinzipen arbeiten, mit welchen zwischen einem Vorhandensein von Gas oder Flüssigkeit im Infusionssystem unterschieden werden kann. Ein Beispiel einer konkreten Ausführungsform wird weiter unten beschrieben.

Die erfindungsgemäße Kassette 1 kann in einer Ausführungsform mit einer zuvor beschriebenen Einstellung oder Regelung des Infusionsdrucks über eine Veränderung des Höhenunterschieds zwischen dem Behälter 30 und der Höhe der Infusionsstelle erfolgen. Dabei kann die erfindungsgemäße Kassette 1 in einer Ausführungsform eine Infusions-Ventil 35 aufweisen, mit welchem der Durchflussquerschnitt eines Flüssigkeitskanals des Infusionssystems veränderbar ist, insbesondere geöffnet oder verschlossen werden kann, optional aber auch eine einstellbare Zwischenstellung einnehmen kann. Ein Beispiel einer konkreten Ausführungsform wird auch hier weiter unten beschrieben.

Bei einer anderen Ausführungsform einer erfindungsgemäßen Kassette 1 kann auch eine aktive Infusion mittels einer aktiven Flüssigkeits-Fördereinrichtung 36 erfolgen. Hierzu kann beispielsweise eine Pumpeinrichtung, wie etwa eine Peristaltikpumpe oder eine andere Fördereinrichtung für Flüssigkeit im Infusionssystem vorhanden sein. Bei einer derartigen Ausführungsform kann optional auch auf eine Infusions-Ventil 35 und/oder eine Höhenverstellung des Infusionsbehälters 30 verzichtet werden. Durch entsprechende Ansteuerung der Fördereinrichtung 36 kann dabei der Infusionsdruck und/oder das Fördervolumen der Infusion variiert werden, insbesondere gesteuert oder geregelt werden.

Eine weitere, bevorzugte Ausführungsform einer erfindungsgemäßen Kassette 1 kann auch - wie in der Figur gezeigt - beides, eine Infusionsbehälter-Höhenverstellung 32 und eine Infusionsflüssigkeits-Fördereinrichtung 36 aufweisen. Dabei kann insbesondere ein Ventil 35 als Bypass zur Fördereinrichtung 36 ausgebildet sein. Somit kann mit derselben Kassette 1 entweder eine aktive (also durch die Fördereinrichtung 36 aktiv initiierte) oder eine hydrostatische (also durch einen Höhenunterschied bewirkte) Infusionsdruck-Variation bereitgestellt werden. Insbesondere kann dabei bei Bedarf zwischen diesen beiden Prinzipien nahtlos - also z.B. auch während des Betriebs - umgeschaltet werden, beispielsweise entsprechend der aktuellen Anforderungen der ausgeführten Operation oder entsprechend den Vorlieben des Chirurgen.

Das erfindungsgemäße Infusionssystem kann zudem eine Infusions-Druckmesseinrichtung 37 aufweisen, mittels welcher ein Druck im Infusionssystem ermittelbar, überwachbar und/oder regelbar ist. Vorzugweise ist diese Infusions-Druckmesseinrichtung 37 nach dem Infusions-Ventil 35 und/oder der Pumpeinrichtung 36 angeordnet, sodass deren ermittelter Druck jenem entspricht, welcher dem Auge zugeführt wird. Da im Infusionssystem gegenüber der Atmosphäre nur Überdruck herrschen kann (oder zumindest sollte), kann diese Infusions-Druckmesseinrichtung 37 derart ausgebildet sein, dass mit dieser nur Überdruck bestimmbar ist, und nicht notwendigerweise auch Unterdruck. Ein Beispiel einer konkreten Ausführungsform wird auch hier im Folgenden beschrieben.

Die Infusionsflüssigkeit wird mittels der erfindungsgemäßen Kassette 1 mit einstellbarem Druck und/oder Fördervolumen an einem externen Infusions-Anschluss 38 der Kassette 1 bereitgestellt, welcher mit einer Leitung oder einem Schlauch hin zum chirurgischen Eingriffswerkzeug verbunden werden kann, sodass die Infusion ins Auge mit einstellbarem und/oder regelbarem Druck und/oder Volumen bereitgestellt werden kann. Um Sicherheit zu gewährleisten, kann dabei der tatsächliche vorherrschende Druck der Infusion hin zum Patienten mit einem entsprechenden Drucksensor überwacht werden.

Vorzugsweise kann dabei erfindungsgemäß der Flüssigkeitspfad in der Kassette 1 in eingelegtem Zustand in Durchflussrichtung substantiell von unten nach oben ausgebildet sein, sodass eine gute Füllung des Aspirationssystems erreicht und eine Ansammeln von Luftblasen vermieden wird.

Hier gezeigt ist auch noch ein erfindungsgemäßer optionaler Pfad vom Infusionssystem hin zum Aspirationssystem der Kassette gezeigt, welcher mit einem Venting-Ventil 39 freigebbar ist. Mit diesem kann beispielsweise, insbesondere bei Inbetriebnahme der Kassette 1, eine Befüllung des Aspirationssystems mit Flüssigkeit erfolgen, und/oder es kann eine Rückspülung von Infusionsflüssigkeit durch das Aspirationssystem durchgeführt werden, beispielsweise zum Lösen von Okklusionen, für eine Reflux-Funktion oder generell zum Unterdruckabbau im Aspirationspfad, z.B. wenn der Arzt via Fußschalter den gewünschten Unterdruck abbauen möchte. Für diese Funktionalitäten können allenfalls noch weitere Ventile zur entsprechenden Schaltung der Flüssigkeitsströme in der Kassette vorhanden sein. Einige Beispiele sind exemplarisch in den folgenden Figuren gezeigt.

Das Aspirationssystem der erfindungsgemäßen Kassette 1 wird über einen Aspirationsanschluss 50 mit einer Leitung oder einem Schlauch ebenfalls hin zum chirurgischen Eingriffswerkzeug verbunden. Auch hier kann wiederum, wie andernorts beschrieben, zumindest ein Luftblasensensor 34b im Aspirationssystem vorhanden sein. In der Figur gezeigt ist auch ein Aspirationsventil 51, welches insbesondere nach den hier angeführten allgemeinen Beschreibungen zu Ventilen ausgebildet sein kann.

Auch erfolgt im Aspirationssystem wiederum eine Druckmessung mit einer Druckmesseinrichtung 10. Bei der Aspiration ergibt sich jedoch die Anforderung, dass mit der Druckmesseinrichtung 10 Unterdrücke gegenüber der Atmosphäre erfassbar sein müssen, vorzugsweise sollten sowohl Unter- als auch Überdrücke erfassbar sein.

Zur Erzielung der Aspirationswirkung zeigt die Figur zwei Varianten. Zum einen eine Peristaltik-Aspiration und zum anderen eine Venturi-Aspiration. Eine erfindungsgemäße Ausführungsform einer Kassette 1 kann entweder nur die Peristaltik-Aspiration aufweisen, oder eine andere erfindungsgemäße Ausführungsform einer Kassette 1 kann nur die Venturi-Aspiration aufweisen oder eine weitere erfindungsgemäße Ausführungsform kann sowohl die Peristaltik-Aspiration als auch die Venturi-Aspiration aufweisen.

Bei der Venturi-Aspiration wird die Aspirationswirkung durch einen Luft-Unterdruck bewirkt, welcher meist mit einer namensgebenden Venturi-Düse erzeugt wird. Jedoch können auch andere unterdruckerzeugende Prinzipien genutzt werden, insbesondere wie diese in den Ausführungsformen der am selben Tag vom demselben Anmelder eingereichten, parallelen Patentanmeldung Nummer EP 16196998 beschreiben sind, welche hiermit per Referenz mit einbezogen werden. Diese Varianten sind hier durch das Venturi-Ventil 57 und das Unterdrucksystem 58 symbolisiert.

Bei der Peristaltik-Aspiration wird eine Flüssigkeits-Fördereinrichtung 52 zur Erzeugung der Aspirationswirkung angewandt. Die Fördereinrichtung 52 kann insbesondere eine Peristaltikpumpe sein, optional ist aber auch z.B. eine Membranpumpe oder eine ähnliche Flüssigkeitsfördereinrichtungen einsetzbar.

Die aspirierte Flüssigkeit wird dabei vorzugsweise in einen entsprechenden Abfall-Behälter 56 gefördert, welcher vorzugsweise an einem entsprechenden Abfall-Anschluss 55 außerhalb der Kassette 1 angeschlossen ist. Dabei kann wiederum ein Drucksensor 37c, insbesondere für Überdruck, genutzt werden, um einen Druckanstieg bei vollem Abfallbeutel zu detektieren. Alternaiv kann auch eine anders ausgestaltete Füllstandsüberwachung des Abfall-Behälters 56 erfolgen. Auch kann, wie hier gezeigt optional auch ein weiter Luftblasensensor 54 im Flüssigkeitskanal zum Abfall-Behälter 56 vorhanden sein. Beispielsweise kann auch bei Fehlfunktionen, Verschmutzung oder wenn während der Beutelentleerung das Ventil 51 nicht komplett dicht ist, dieses mit dem Drucksensor 10 patientenseitig detektiert werden und es können entsprechende Sicherheitsmassnahmen eingeleitet werden.

Die erfindungsgemäße Kassette 1 kann auch eine, insbesondere mechanische und/oder optische, Codierung 70 aufweisen, welche von einer entsprechenden Leseeinheit 71 des Geräts 99 erfasst und ausgewertet werden kann. Anhand dieser Codierung kann - beispielsweise bei Einwegkassetten - ein mehrfache oder nichtsterile Nutzung verhindert werden, eine spezifische Ausführungsform der eingelegten Kassette und deren optionalen Funktionalitäten, etc. vom Gerät 99 erkannt werden. Beispielsweise kann es sich in einer Ausführungsform um einen aufgedruckten, eingravierten oder laserbeschrifteten Code 70 handeln, speziell in Form eines Barcodes oder eines QR-Codes 70, welcher von einer optischen Erfassungseinheit 71, wie z.B. einer Kamera 71 im Gerät 99 beim Einlegen oder bei eingelegter Kassette 1 erfasst wird.

**Fig. 2** zeigt eine weitere beispielhafte Ausführungsform einer erfindungsgemäßen Kassette 1 in einem Blockdiagramm. Die zu Fig. 1 funktional gleichen oder gleichwertigen Merkmale sind dabei mit ähnlichen Bezugszeichen versehen und es wird auf deren obenstehende Beschreibung verwiesen.

Die wesentlichen Unterschiede dieser erfindungsgemäßen Ausführungsform zu Fig. 1 sind erfindungsgemäß, dass im Infusionsbereich lediglich eine aktive Infusion mittels einer Fördereinrichtung 36 erfolgt. Das Infusionsventil 35 ist hier optional und kann z.B. als Absperrung gegenüber dem Patienten ausgebildet sein um zusätzliche Sicherheitsfunktionen zu realisieren oder mit dem optionalen Ventil 39 zusammenzuwirken. Im Aspirationsbereich ist erfindungsgemäß der Abfallbeutel 56 direkt an der Kassette 1 anbringbar, es ist ein zweiter, optionale Aspirationsanschluss 50b mit entsprechendem zweitem Aspirationsventil 51b gezeigt. Optional sind beispielsweise zwischen Patient und Pumpeinrichtung 52 bzw. 36 aktive oder passive Ripple-Kompensatoren 59a und/oder 59b zum Ausgleich von Druck- oder Fördermengen-Ripple der Peristaltikpumpe 52 bzw. 36 angeordnet. Verschiedene Aspekte aus Fig. 1 und Fig. 2 können erfindungsgemäß für unterschiedliche erfindungsgemäße Ausführungsformen auch vertauscht und/oder vermischt werden.

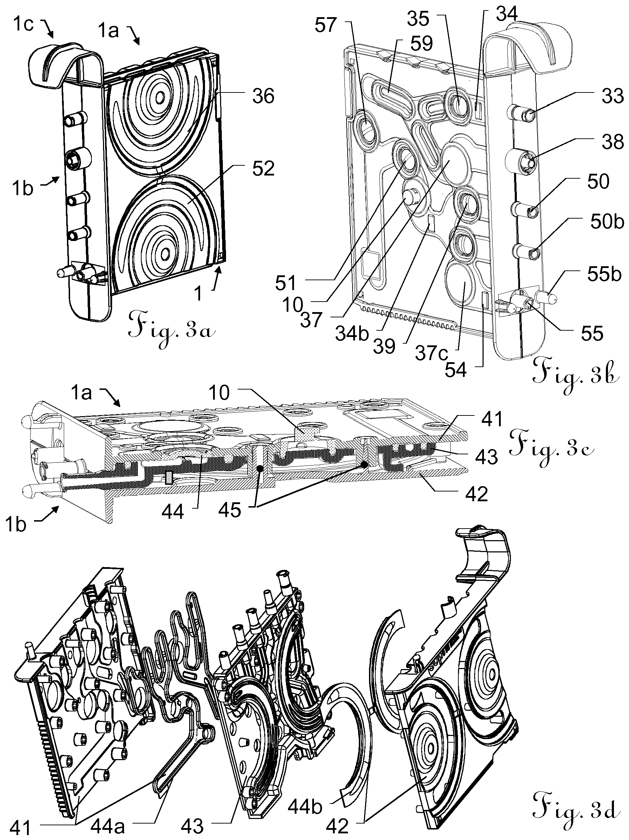

In **Fig. 3a** und **Fig. 3b** ist eine weitere Ausführungsform einer erfindungsgemäßen Kassette 1 jeweils in einer Ansicht von rechts vorne(Fig. 3a) und links vorne (Fig. 3b) dargestellt. Die Kassette 1 ist in diesem Beispiel als eine, in ihren Außenkonturen zumindest annähernd quaderförmiger Basis-Kassette 1a mit einer überragenden Platte als Frontpartie 1b gezeigt. Eine derartige Formgebung ermöglicht eine platzeffiziente Lagerung und Stapelbarkeit der Kassette 1, insbesondere bei Aufbewahrung, Versand, etc. Die gezeigte Frontpartie 1b, welche einen Griff 1c ausbildet, ermöglicht eine gute Handhabbarkeit bei der Einführung in das Gerät 99, insbesondere unter Wahrung der Sterilität der relevanten Teile der Kassette 1 und vor allem keine Kontamination zwischen Gerät 99 und dem Personal im sterilen Bereich. In dieser Ausführungsform sind an der Frontpartie 1b auch alle Anschlüsse für Schlauchsysteme zu finden, insbesondere jene an das Operations-Handstücks, an die Infusionsflüssigkeits-Reservoir 30, an einen Abfallbeutel 56, etc. Vorzugsweise sind diese Anschlüsse 33, 38, 50, 50, 50b, 55 mechanisch und/oder geometrisch codiert, um die Gefahr eines fälschlichen Anschließens und Verwechslungen auszuschließen. Diese Codierung kann neben optischer Kennzeichnung auch durch unterschiedliche geometrische Ausformung der Schale 41,42 und/oder des Kernteils der Kassette 1 erfolgen, durch welche diese Anschlüsse gebildet werden. Die während des Einbringens und/oder des Betriebs von außen zugängliche Frontpartie 1b der erfindungsgemäßen Kassette 1, kann also entsprechend eines Aspekts der Erfindung derart ausgebildet sein, dass diese einen Handgriff 1c ausformt, welcher eine Manipulation der Kassette 1 ermöglicht, ohne dabei mit dem unsterilen Gerät in Berührung zu kommen. Die Frontpartie 1b kann auch als in Einbringrichtung wirkender Positionsanschlag beim Einbringen der Kassette 1 in das Gerät 99 dienen.

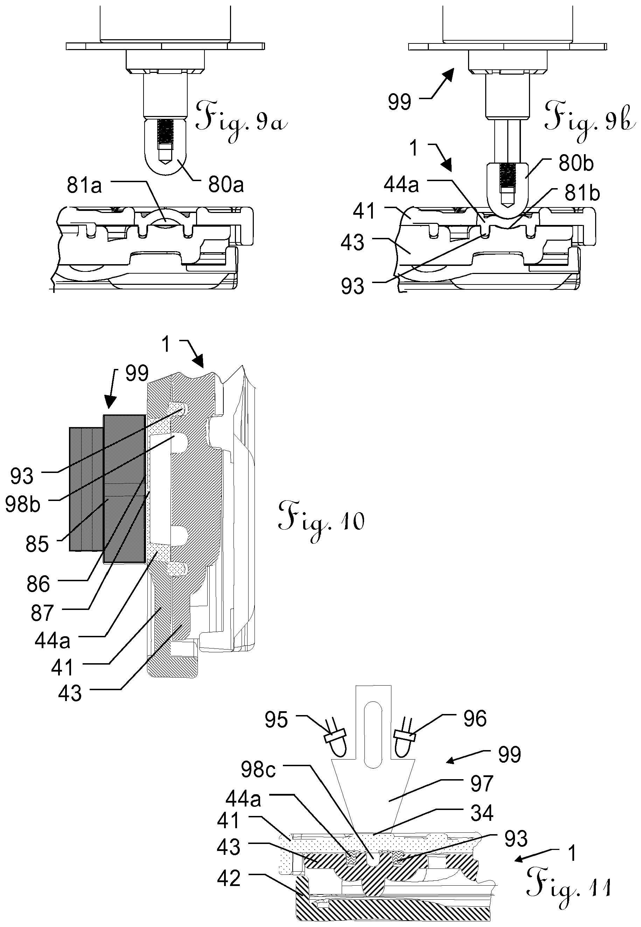

In dieser Figur ebenfalls zu erkennen sind die optionalen Luftblasen-Detektoren 34, 34b und 54. Diese sind in der hier gezeigten Ausführungsform optisch wirkend ausgebildet. Beispielsweise können diese auf dem Prinzip von unterschiedlichen Brechungsindizes von Flüssigkeit und Luft basierend ausgebildet sein, insbesondere kann ein unterschiedlicher Winkel der Totalreflexion zwischen Flüssigkeit und Luft, oder unterschiedliche Lichtleiteigenschaften von Flüssigkeit und Luft, oder eine anderes Wirkprinzip genutzt werden, beispielsweise ein kapazitives. Vorzugsweise befinden sich diese Luftblasendetektoren 34 zumindest annähernd im Nahbereich der zugehörigen Anschlüsse 33,38, 50,50b und/oder 55. Beispielsweise kann ein solcher Luftblasen-Detektor 34,54 mit einem, zumindest in der genutzten Wellenlänge optisch transparenten, in einer Außenschale 41 und/oder 42 implementierten, Sichtfenster in den Flüssigkeitskanal ausgebildet sein. In einer Ausführungsform kann beispielsweise die Außenschale aus transparentem Material ausgebildet sein. Fig. 11 illustriert eine beispielhafte erfindungsgemäße Ausführungsform.

Vorzugsweise ist nach einem Teilaspekt der Erfindung, die Anordnung und Ausbildung der Flüssigkeitskanäle innerhalb der Kassette 1 derart, dass bei einem Füllen der Flüssigkeitskanäle mit Flüssigkeit, dieses Füllen stets (zumindest substantiell) von unten nach oben erfolgt - womit allfällige Luftblasen (dem Mediumsdichtenverhältnis entsprechend) nach oben verdrängt und vom Patienten weg abgeführt werden. Ein derartiges Füllen der Flüssigkeitskanäle der Kassette erfolgt dabei insbesondere nach dem Einlegen der Kassette 1 bei der Inbetriebnahmen der Kassette 1. Entsprechend kann entsprechend dieses Aspekts der Erfindung die Anordnung der Anschlüsse von unten nach oben in der Reihenfolge Abfallbeutel 55 - Aspiration-A vom Patienten 50 - ev. Aspiration-B 50b - Infusion zum Patienten 38 - Infusionsflasche 33 ausgebildet sein.

Im gezeigten Beispiel von Fig. 3c ist die im Schnitt dargestellte Kassette 1 im wesentlichen dreiteilig aufgebaut, mit einer linken Außenschale 41, einer rechten Außenschale 42 und einem Kernteil 43. Vorzugsweise sind die Außenschalen 41 und 42 gegeneinander und/oder gegen das Kernteil 43 verschnappbar, verpressbar oder verquetschbar ausgebildet, also beispielsweise mit einem Hakensystem, einem Press-Fit System oder ähnlichem, welche insbesondere nicht wieder lösbar ausgestaltet sein können. In der Figur sind beispielhaft Pressfit-Verbindungen 45 gezeigt. In einer andern Ausführungsform kann die erfindungsgemäße Kassette 1 allenfalls auch nur zweiteilig konstruiert sein, also insbesondere ohne separates Kernteil 43, wobei die kassetteninterne Flüssigkeitskanäle von den beiden Außenschalen 41 und 42 gebildet werden können.

Zudem ist in dieser Schnittdarstellung ein Teil der Funktionselemente gezeigt. Dabei ist der Weichkunststoff-Anteil der 2K-Spritzguss-Außenschalen 41 und 42 mit dem Kernteil 43 verspannt und/oder formschlüssig im Eingriff, sodass dabei die Flüssigkeitskanäle bzw. Leitungen innerhalb der Kassette gebildet werden. Im gezeigten Schnitt bildet der Weichkunststoff entlang der Flüssigkeitskanäle Wülste aus, welche in zusammengebautem Zustand in Vertiefung des Kernteils 43 eingreifen - womit eine Flüssigkeitsabdichtung bewirkt wird, insbesondere durch Quetschung des Weichkunststoffs und/oder Ausbildung einer Mäanderdichtung mit zumindest einer einzigen Abstufung. Teilbereiche des Weichkunststoff-Anteils sind dabei in montiertem Zustand der Kassette 1 von außen zugänglich und bilden die Funktionselemente aus. Der Hartkunststoff-Anteil der 2K-Spritzguss-Außenschalen 41 und 42 weist dafür entsprechende Ausschnitte auf.

In der hier gezeigten Ausführungsform sind die Außenschalen 41 und 42 als einstückige 2-Komponenten-Spritzgussteile aus einem Hartkunststoff und einem Weichkunststoff 44 ausgebildet. Beispielsweise mit einem Polyethylen, Polyamid oder einem Polypropylen oder einem anderen für derartige medizinische Anwendungen geeigneten und zugelassenen steifen Kunststoff als Hartkunststoff, sowie beispielsweise einem Silikon oder einem, insbesondere thermoplastischen, Elastomer, oder einem anderen für derartige medizinische Anwendungen geeigneten und zugelassenen WeichKunststoff 44. Das Kernteil 43 ist ebenfalls aus einem Hartkunststoff ausgebildet, beispielsweise einem zuvor genannten. Eine allfällige Sterilisierbarkeit der gesamten Kassette, beispielsweise im Autokalven, etc. kann bei der Materialwahl der Kassettenmaterialen ebenfalls Berücksichtigung finden, insbesondere falls eine mehrfach verwendbare Ausführungsform angestrebt wird.

**Fig. 3d** zeigt eine Explosionsdarstellung einer beispielhaften erfindungsgemäßen Kassette 1, wobei der Übersichtlichkeit halber auch die Weichkunststoff-Bereiche 44a und 44b getrennt von dem jeweils zugehörigen Hartkunststoffteil 41 bzw. 42 dargestellt sind - obgleich diese fertigungstechnisch einstückig als 2K-SpritzgussTeile ausgebildet sein können. Im gezeigten Beispiel bestehen die Weichkunststoff-Abschnitte der Außenschalen 41 und 42 des 2K-Spritzgussen aus einem thermoplastischen Elastomer (TPE) und sind derart ausgeformt, dass diese gemeinsam mit dem Kernteil 43 die Flüssigkeitskanäle innerhalb der Kassette 1, sowie die funktionalen Elemente (wie z.B. Pumpabschnitte, Ventile, Drucksensoren) ausbilden. Speziell kann eine Ausführungsform mit 2K-Spritzguss-Außenschalen 41 und 42 aus Hartkunststoff mit Weichkunststoff gebildet sein, und einem 1K-Kernteil 43 aus nur Hartkunststoff. Wenn in einer derartigen Ausführungsform lediglich Bereiche des Kernteils und Bereiche des Weichkunststoffs indirekt über die Flüssigkeiten mit dem Patienten in Berührung kommen, jedoch keine Teile der Außenschalen 41 und 42, sind auch nur bezüglich der Kernteil- und Weichkunststoff-Materialien allfällige medizinische Materialrichtlinien einzuhalten, wohingegen die Materialwahl bei den Außenschalen 41 und 42 diesbezüglich flexibler, und z.B. primär den Festigkeits- und Ästhetik-Anforderungen angepasst, gewählt werden kann.

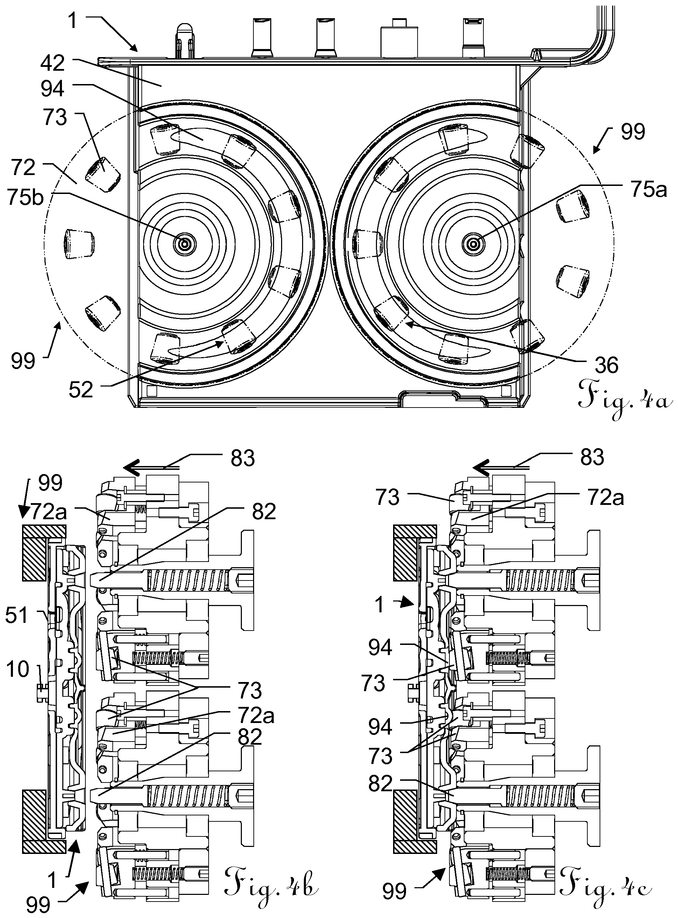

In den in Fig. 3a, Fig. 3b, Fig. 3c und Fig. 3d, dargestellten Beispielen von Ausführungsformen sind die funktionalen Elemente jeweils auf beide der gezeigten Außenschalenhälften 41 und 42 aufgeteilt, womit ein kompakter Aufbau erzielbar ist. Dabei sind im gezeigten Beispiel speziell auf einer Kassettenseite 42 die Quetschpumpen-Abschnitte, und auf der anderen Kassettenseite 41 die Ventile und Drucksensoren angeordnet. Eine derartige Aufteilung der funktionalen Elemente ist nicht zwingend, kann aber bezüglich einer Einspannung oder Klemmung der Kassette 1 im Gerät 99 vorteilhaft sein, bei welcher mit dem Anpressen des oder der Rollenköpfe der Peristaltikpumpe zugleich eine Klemmung, Fixierung und/oder Fein-Positionierung der Kassette 1 im Gerät 99 erfolgen kann. Fig. 4b und Fig. 4c illustriert eine beispielhafte Ausführungsform einer erfindungsgemäßen Peristaltikpumpe einer erfindungsgemäßen Kassette 1. Dabei kann die zur Klemmung der Kassette im Gerät 99 erforderliche Bewegung nur von einer Seite, vorzugsweise von der Seite der Peristaltik-Rollen 73 erfolgen. Beispielsweise können hierbei Schrägflächen, z.B. im Bereich des Drehzentrums 75 der Peristaltik-Rollen oder auch anderswo an der Kassette 1 und/oder an der Klemmeinrichtung des Geräts 99 ausgebildet sein, welche nach dem Einlegen der Kassette 1 mit entsprechenden geräteseitigen Pendants eine Positionierung und Fixierung der Kassette 1 innerhalb des Geräts 99 bewirken.

**Fig. 4a** zeigt schematisch ein solches Klemmen und fixieren der eingeschobenen Kassette 1 im Gerät 99 in einer Seitenriss-Darstellung, mit Blickrichtung auf Peristaltikpumpen der Kassette 1. Dabei ist ein geräteseitiger 99 Rollenkopf 72 mit Rollen 73 gezeigt, welche Rollen 73 auf dem aus Weichkunststoff 44b gebildeten, wulstförmig aufgewölbten Quetschbereich 94 der Außenschale 42 abrollen. Bei diesem Abrollen wird der Quetschbereich 94 von den Rollen 73 abdichtend auf das Kernteil gedruckt und bei einer Drehung des Rollenkopfes 72 und das Drehzentrum 75 eine Förderung eines Volumens im Quetschbereich 94 zwischen den Rollen 73 bewirkt.

**Fig. 4b** ist die Kassette 1 in Einlegerichtung orthogonal zur Zeichnungsebene in das Gerät 99 bis zu seiner Endlage eingelegt, aber noch nicht geklemmt. Es handelt sich um eine Schnittdarstellung durch das Rechte und linke Drehzentrum 75 der hier gezeigten zwei getrennten Peristaltikpumpen 36 und 52. In diesem Beispiel einer erfindungsgemäßen Ausführungsform wird nach dem Einlegen das Klemmen der Kassette 1 gemeinsam mit einem Andrücken der Rollenköpfe 72 der Peristaltikpumpe in Richtung 83 bewirkt. Wie gezeigt kann dabei die Klemmkraft der Rollen 73 und/oder der Positionsausrichtung 82 mittels Federn bestimmt werden.

Ebenfalls erfindungsgemäß im Gerät 99 angeordnet sind die hier nicht gezeigten Aktoren, welche auf die Ventile 35,39,51,51b und/oder 57, einwirken. Diese können beispielsweise als elektromagnetisch oder elektromotorisch oder pneumatisch betätigte, bewegliche Stößeln im Gerät ausgebildet sein, welche mechanisch auf die Ventilausformungen der Kassette 1 einwirken. Dabei kann ein Durchflussquerschnitt eines Abschnitts der Flüssigkeitskanäle in der Kassette 1 verändert werden, insbesondere freigegeben oder blockiert werden. Fig. 9a und Fig. 9b illustriert eine beispielhafte Ausführungsform.

An der Kassette 1 ist auch eine erfindungsgemäße Druckerfassungseinrichtung 10, speziell dessen Kupplungselement 49 gezeigt, welches im eingelegten Zustand der Kassette 1 mit dem hier nicht gezeigten, geräteseitigen Kraftsensor 11 zusammenwirkt. In der gezeigten Ausführungsform ragt das Kupplungselement 49 des Kraftsensors 11 über die Außenschale 41 hinaus und kann beim einlegen beispielsweise entlang einer geräteseitigen Nut in Einlegerichtung bis zur Kopplung mit dem Kraftsensor 11 eingeführt werden. Bei einer erfindungsgemäßen Druckerfassungseinrichtung 10, welche in ihrer Funktion gegenüber dem Atmosphärendruck nebst Überdruck auch Unterdruck feststellen muss, ist eine negative - also bezüglich der Kassettenhülle nach Innen gerichtete - Kraft auf die Membrane zu detektieren. Daher ist das zuvor bei einem reinen Überdrucksensor 37 beschriebene, einfache anliegen einer Membrane an einer Sensitivitäts-Fläche eines Kraftsensors, nicht hinreichend, da hierbei nur bezüglich der Kassettenhülle nach Außen wirkende Kräfte erfassbar sind.

Eine erfindungsgemäße Druckerfassungseinrichtung 10 in einer Kassette 1, besonders wenn diese 2K-Spritzgussteile aufweist, hat gegenüber dem Stand der Technik eine vielzahl von Vorteilen, beispielsweise einen weniger komplexen Aufbaus mit weniger Teilen, eine Vereinfachung des Herstellungsprozesses der Kassette, weniger Produktionsmaschinen welche unter sterilen Bedingungen arbeiten müssen, keine oder weniger aufwändigen Kontrollen von Schweiß- oder Klebenähten, geringeren Montageaufwand bei der Kassettenfertigung, vereinfachte Handhabung der Kassette 1 beim Einlegen, zuverlässige Kopplung der Druckerfassungseinrichtung 10, automatische Kopplung der Druckerfassungseinrichtung 10 durch einfaches lineares einschieben der Kassette 1, anbringbarkeit von optionalen Zusatzmodulen an der Kassette oder variantenvielfältige Kassetten - da sämtliche Eingriffe der Funktionselemente der Kassette 1 seitlich erfolgen und die Kassette in Einschubrichtung in ihrer Länge variieren kann, vorteilhaftes Packungsformat zur kompakten Lagerung der Kassetten 1, und vieles mehr.

**Fig. 4c** zeigt die Kassette 1 in geklemmtem Zustand im Gerät, in welchem alle Sensoren und Aktoren des Geräts 99 mit ihren Gegenstücken der Kassette 1 zur Interaktion korrespondieren. Im gezeigten Beispiel erfolgt dieses durch ein Klemmen der Kassette 1 im Gerät 99, welches durch andrücken der Rollenköpfe 72 an die Kassette in Richtung 83 erfolgt, womit die Kassette 1 in der Zeichnungsebene nach links auf entsprechende mechanische Anschläge gedrückt wird. Dabei Quetschen die Rollen 73 die Quetschkanäle 94 - wobei bei der oberen Pumpe (z.B. der Infusionspumpe 36) ein Schnitt durch die Rolle 73 mit gequetschtem Quetschquerschnitt 94 sowie bei der unteren Pumpe (z.B. der Aspirationspumpe 52) ein Schnitt neben einer Rolle 73 und entsprechend einen ungequetschten Quetschquerschnitt 94 zeigt. Mit den Zentrierelementen 82, welche in diesem Beispiel auch die Drehachse 75 des Rollenkopfes 72 bilden wird dabei beim Klemmen der Kassette 1 auch eine Fein-positionierung der Kassette 1 im Gerät 99 bewirkt.

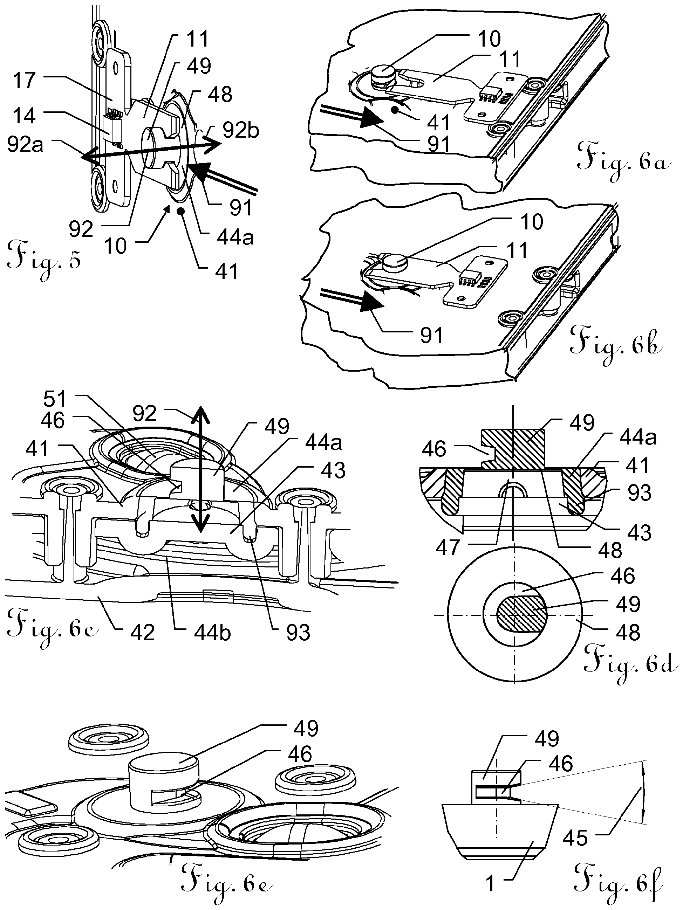

**Fig. 5** illustriert nun einen Ausschnitt eine beispielhafte Ausführungsform einer erfindungsgemäßen Kassette 1 welche im Gerät 99 eingelegt ist. Dabei befindet sich die Gabel 11 des erfindungsgemäßen geräteseitigen Kraftsensors mit dem erfindungsgemäßen kassettenseitigen Kupplungselement 49 der Druckerfassungseinrichtung 10 im Eingriff. Sowohl positive 92a also auch negative 92b, von den Druckverhältnissen in der Kassette 1 ausgeübte Kraftwirkungen 92 auf die Membrane 48 sind somit übertragbar. Die Kraftwirkung 92 ist hier zumindest annähernd normal zur Fläche der Membrane 48 und auch zumindest annähernd normal zur Einschubrichtung 91. Dabei weist der gezeigte Ausleger, welcher die Gabel 11 ausbildet, zumindest einen Teil der Messelektronik 14 auf, insbesondere eine erste Verstärkerstufe, mittels welcher die Kraftwirkung 92 zur Erfassung als elektrisches Signal bereitgestellt wird. In dieser Ausführungsform ist eine Kraftmessung mittels Dehnmessstreifen gezeigt, welche an einer Stelle des Auslegers, welche eine verringerte Querschnittsfläche aufweist, angeordnet sind. Vorzugsweise ist dabei auch zumindest eine erste elektronische Verstärker- und/oder Auswertestufe direkte auf dem oder beim Ausleger angeordnet, speziell um elektrische Störeinflüsse zu vermeiden. Der hintere Abschnitt 17 des Auslegers ist fest im Gerät 99 verankert.

Die erfindungsgemäße Herstellung der Verbindung zur kassettenseitigen Druckerfassungseinrichtung 10 kann in der gezeigten Ausführungsform in Einschubrichtung 91 der Kassette 1 in das Gerät 99 erfolgen, indem beim Einschieben der Kassette 1 in das Gerät 99, die Gabel 11 der geräteseitigen Druckerfassungseinrichtung 10 mit dem nippelförmigen Kupplungselement 49 der kassettenseitigen Druckerfassungseinrichtung 10 in Eingriff gebracht wird. Dabei greift die Gabel 11 in die Ausnehmung 46 des Kupplungselements 49 ein, welche Ausnehmung 46 auch als Schlitz 46 oder Einkerbung 46 bezeichnet werden könnte. Die Gabel ist dabei substantiell parallel zur Membrane 48 ausgerichtet und die Gabel 11 umfasst das Kupplungselement 49 an der Stelle der Ausnehmung 46 zumindest Teilweise - vorzugsweise zumindest an zwei gegenüberliegenden Seiten des Kupplungselements 49 in der Ausnehmung 46. Dabei können Gabel 11 und/oder Ausnehmung 46 vorzugsweise auch mit einer Anschrägung ausgebildet sein, insbesondere um damit beim Einführen größere Tolleranzen der ineinander eingreifenden Bereiche zueinander zu gestatten, sodass die Kassette 1 um Gerät 99 weniger exakt geführt werden muss.

**Fig. 6a** zeigt ein Beispiel einer erfindungsgemäßen Ausführungsform, bei welcher die Kassette 1 nur teilweise in ins Gerät 99 eingeschoben ist. Die Einschubrichtung 91 stellt dabei die Bewegung der Kassette 1 gegenüber dem im Gerät 99 feststehenden Kraftsensor 11 dar. Dabei ist die Gabel des Kraftsensors 11 an der vorderen, flachen Zunge des Auslegers des Kraftsensors kurz vor dem Eingreifen in die kassettenseitige Druckerfassungseinrichtung 10 - die Kassette 1 ist also beinahe vollständig eingeschoben. Die Zinken der Gabel 11 sind gerade im Begriff in Einschubrichtung in die Ausnehmung 46 des Kupplungselements 49 einzugreifen. Die Membranenfläche 48 der Druckkammer 47 der Kassette 1 ist dabei (zumindest annähernd - also marginale toleranz- oder spielbedingte Abweichungen sind möglich) parallel zur Einschubrichtung 91 angeordnet. Ebenso ist die Gabel 11 des geräteseitigen Auslegers (zumindest annähernd) parallel zur Einschubrichtung 91 und somit auch (zumindest annähernd) parallel zur Membrane 48 angeordnet. Entsprechend ist erfindungsgemäß auch die Ausnehmung 46 im Kupplungselement 49, in welche die Gabel 11 zumindest zweiseitig in das Kupplungselement 49 eingreift, substantiell parallel zur Einschubrichtung 91 der Kassette 1 ausgebildet.

**Fig. 6b** zeigt ein Beispiel einer erfindungsgemäßen Ausführungsform, bei welcher die Kassette 1 vollständig eingeschoben ist - die Kassette 1 befindet sich also in der, für deren Betrieb vorgesehenen Endlage. Dabei ist die Gabel 11 des geräteseitigen Kraftsensors 14 erfindungsgemäß im Eingriff mit dem Kupplungselement 49 der kassettenseitigen Druckerfassungseinrichtung 10, und es kann eine erfindungsgemäße Über- und Unterdruckmessung erfolgen. In gezeigten Beispiel sind bei eingelegter Kassette 1 beim Kupplungselement 1 nicht nur seitlich die Zinken der Gabel 11 im Eingriff, sondern es ist auch der hintere Teil des gabelförmigen, flachen Auslegers 11, welcher sich zwischen den Zinken befindet, im Eingriff mit der Ausnehmung 46 des Kupplungselements 49 - das erfindungsgemäße Kupplungselement 49 dieser Ausführungsform ist also derart ausgebildet und angeordnet, dass durch die Gabel 11 in das Kupplungselement 49 über einen Winkelbereich von mehr als 180° eingegriffen werden kann, was vorteilhaft bezüglich der zu bewirkenden Kraftübertragung von der Membrane 48 auf den Kraftsensor sein kann, insbesondere da dies eine zuverlässige Kopplung bewirkt.

**Fig. 6c** und **Fig. 6d** zeigen eine beispielhafte Ausführungsform eines erfindungsgemäßen Kupplungselements 49 einer Druckerfassungseinrichtung 10 in Schnittdarstellung. Zur Linken ist in Fig. 6c eine dreidimensionale Darstellung gezeigt, bei welcher eine Außenschale 41 der Kassette 1 aus Hartkunststoff mit einem Weichkunststoff 44a als ZweiKomponenten-Spritzguss-Teil hergestellt wurde. Der Weichkunststoff 44a bildet dabei Funktionselemente, speziell die hier gezeigte Druckerfassungseinrichtung 10, sowie Dichtungen 93 der kassetteninternen Flüssigkeitskanäle 47 dar. Im Hintergrund ist ein Ventil 51 zu sehen, welches ebenfalls Teilweise aus dem Weichkunststoff 44a ausgebildet ist. Diese Flüssigkeitskanäle 98 werden von der Außenschale 41 und dem Kernteil 43 gebildet, welches ebenfalls aus Hartkunststoff besteht. Im gezeigten Schnitt bildet ein Bereich eines der Flüssigkeitskanäle 98 eine Druckkammer 47 der Druckerfassungseinrichtung 10 aus. Diese Druckkammer 47 ist mit einem der Flüssigkeitskanäle 98 verbunden, bzw. wird von einem der Flüssigkeitskanäle 98 durchflossen - was hier z.B. mit dem Einlass im Hinteren Bereich der Druckkammer 47 dargestellt ist. Die beispielhaft gezeigte Druckkammer 47 hat einen Boden aus dem Hartkunststoff des Kernteils 43, welcher mit dem Weichkunststoff 44a der Außenschale 41 - abgesehen von einer Verbindung zu den Flüssigkeitskanälen 98 - rundum durch den Weichkunststoff 44a abgedichtet ist - indem der Weichkunststoff 44a eine Dichtung 93 ausbildet.

Der Weichkunststoff 44a bildet ebenfalls den oberen Deckel der Druckmesskammer 47 aus, welcher zumindest Teilweise als flexible Membrane 48 ausgebildet ist. Von der Membrane 48 ragt (gegenüber der Druckkammer 47) nach Außen hin das Kupplungselement 49 auf. In diesem Beispiel ist das Kupplungselement 49 ebenfalls aus dem Weichkunststoff 44a ausgebildet, weist jedoch durch seine massive Ausführung nur eine geringe Elastizität auf - insbesondere eine wesentlich geringere Elastizität als die Membrane 48. Die Membrane 48 umfängt dabei das Kupplungselement 49 umlaufend. Vorzugsweise ist das Kupplungselement 49 also zumindest annähernd zentral in der Membrane 48 angeordnet und das Kupplungselement 49 ist von der flexiblen Membrane 48 umgeben und mittels dieser mit der Kassetten-Außenschale 41 einstückig, aber flexibel verbunden. Bei der Druckmessung wirkt nun der, in der Druckkammer 47 vorherrschende Druck auf die Außenwände der Druckkammer 47 und somit auch auf die Innenfläche des Deckels der Drucckammer 47. Flächeninhalt mal Druck ergeben dabei in bekannter Weise eine Kraftwirkung 92, welche je nach Vorzeichen des Drucks relativ zum Atmosphärendruck nach Innen oder Außen wirkt. Da die Membrane 48 flexibel ist, wirkt diese Kraftwirkung 92 auf das Kupplungselement 49. Mittels einem, mit dem Kupplungselement 49 gekoppelten, geräteseitigen Kraftsensor 14 ist somit über diese Kraftwirkung 92 ein Wert des Flüssigkeitsdrucks in positive und negative Richtung erfassbar. Die Wände der Druckkammer sind hier fertigungstechnisch bedingt mit einer Schicht Weichkunststoff 44a über dem Hartbereich 41 ausgebildet, sodass die umlaufende Dichtung 93 der Druckkammer und die Membrane 48 einstückig ausgebildet sind - dies ist jedoch nicht zwingend erforderlich.

Das Kupplungselement 49 ist dabei derart ausgebildet, dass beim einlegen der Kassette 1 in das Gerät 99 ein geräteseitiger Ausleger eines Kraftsensors 14 selbsttätig in das Kupplungselement 49 eingreift, sodass die Kraftwirkung 92 von der Druckkammer 47 in positiver sowie in negativer Richtung auf den Kraftsensor 14 übertragbar ist. Das Eingreifen erfolgt dabei automatisch beim einlegen der Kassette 1, sodass für die Kopplung von Kupplungselement 49 und Kraftsensor 14 keine manuellen Manipulationen erforderlich sind. Im gezeigten Beispiel wird die Kassette 1 in einen dafür im Gerät 99 vorgesehenen Schlitz eingeschoben - und dabei wird automatisch Kupplungselement 49 mit Gabel 11 des Kraftsensors gekoppelt. Neben einem gänzlich händischen einschieben, kann das Einlegen der Kassette 1 dabei auch zumindest teilweise automatisiert erfolgen, beispielsweise indem ein motorisiertes Einziehen einer in den Schlitz des Geräts 99 gesteckten Kassette 1 erfolgt. Beim Einlegen wird die Kassette 1 in einer vorgesehenen Betriebslage im Gerät 99 positioniert, sodass die Funktionselemente der Kassette 1 mit den korrespondierenden Sensoren und/oder Aktoren des Geräts 99 in ihrer Lage übereinstimmen um ihre Funktion zu erfüllen. Dieses Positionieren kann beispielsweise mit Führungsschienen, mechanische Anschläge, Positionssensoren, und/oder anderen hierzu geeigneten Einrichtungen erfolgen.

Zum Koppeln mit dem Kraftsensor 14 ist das Kupplungselement 49 dabei mit einer Ausnehmung 46 ausgebildet, welche als zumindest eine, zumindest teilweise umlaufende Ausnehmung im Kupplungselement 49 ausgebildet ist. Diese Ausnehmung 46 ist dazu ausgebildet, dass beim Einschieben der Kassette 1 in das Gerät 99 ein gabelförmiger Ausleger 11 des Kraftsensors 14 eingreift. Dieses Eingreifen erfolgt dabei zumindest an zwei gegenüberliegenden Seiten des Kupplungselements 49. Die Ausnehmung 46 kann also beispielsweise als zwei gegenüberliegende seitliche Schlitze ausgebildet sein, in welche zwei Zinken des gabelförmigen Auslegers 11 eingreifen. Alternativ kann eine einzige Ausnehmung 46 zumindest teilweise Umlaufend, also insbesondere über mehr als 180° des Kupplungselements 49, ausgebildet sein, sodass nicht nur die Zinken der Gabel 11, sondern auch die hintere Verbindung zwischen den Zinken in die Ausnehmung 46 des Kupplungselements 49 eingreifen können.

Zur Rechten ist in Fig. 6d analog zu obiger Beschreibung eine Schnittdarstellung einer beispielhaften Konstruktionszeichnung dargestellt, in welcher einige der zuvor beschriebenen Details allenfalls besser zu erkennen sind.

**Fig. 6e** und **Fig. 6f** zeigen eine weitere Darstellung einer beispielhaften Ausführungsform einer erfindungsgemäßen kassettenseitigen Druckerfassungseinrichtung 10, speziell des Kupplungselements 49 der Druckerfassungseinrichtung 10. In der linken Abbildung 6e ist wiederum eine 3D-Darstellung und in der rechten 6f eine Konstruktionsdarstellung desselben gezeigt. Das erfindungsgemäße Kupplungselement 49 hat in diesem Beispiel eine Ausnehmung 46, welche sich über drei Seiten des Kupplungselements 49 erstreckt. Zur Verbesserung der Stabilität ist in dieser Ausführungsform eine vierte Seite des Kupplungselements 49 ohne Ausnehmung 46 ausgeführt, da auf dieser Seite die Gabel 11 nicht eingreifen kann - alternativ kann die Ausnehmung 46 aber auch vollständig umlaufend sein, speziell wenn die Steifigkeit des Kupplungselements 49 für diese Anwendung dann noch hinreichend ist.

In der gezeigten Ausführungsform weist die Ausnehmung 46 eine Anschrägung 45 auf, welche den Fangbereich für den Ausleger 11 des Kraftsensors 14 beim einschieben erhöht und somit das Koppeln erleichtert. Zusätzlich oder alternativ können entsprechend auch die Zinken des gabelförmigen Auslegers 11 des Kraftsensors 14 an ihren Enden eine Anschrägung oder Fase aufweisen, wie dieses etwa in Fig. 5 sowie Fig. 6a und Fig. 6b zu sehen ist. Damit kann der Fangbereich beim Kopplungsvorgang weiter erhöht und das Koppeln beim Einschieben der Kassette 1 zuverlässiger gestaltet werden.

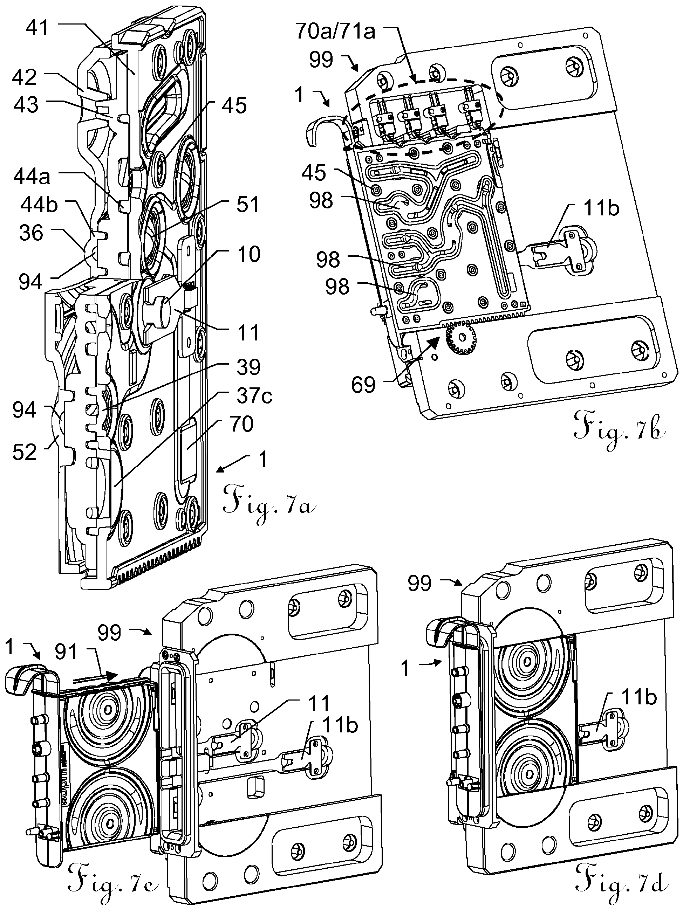

**Fig. 7a** zeigt eine beispielhafte Ausführungsform einer erfindungsgemäßen Kassette 1 in welcher die erfindungsgemäße kassettenseitige Druckerfassungseinrichtung 10 mit dem geräteseitigen Ausleger des Kraftsensors 11, sowie Abschnitte zweier Peristaltikpumpen 36 und 52, Überdrucksensoren 37c und Ventile 39 und 51, sowie eine Vielzahl von Pressfit-Verbindern 45 beispielhaft gezeigt sind.

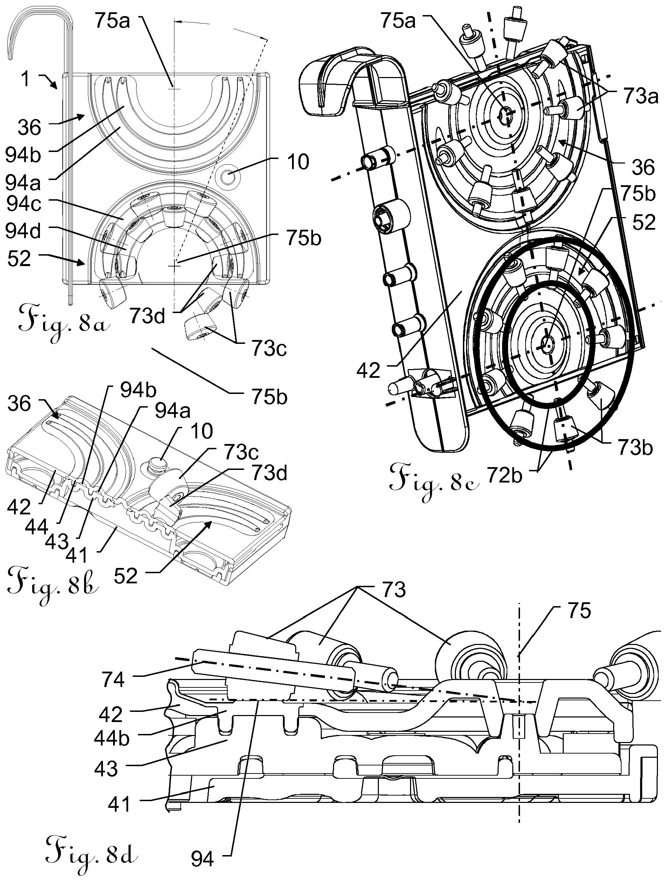

Die Flüssigkeitskanäle 98 im Bereich der Peristaltik-pumpe 36 oder 52 sind im Inneren der Kassette 1 durch den Hartkunststoff-Kernteil 43 gebildet, über welchen ein zum Äusseren der Kassette 1 hin aufgewölbter Weichkunststoffbereich 44b der Außenschale 42 einen Teilbereich des Flüssigkeitskanals 98 ausbildet. Dieser Teilbereich, welcher auch als Quetschbereich 94 der Pumpe bezeichnet wird, ist durch eingreifende, geräteseitige Roller 73 in der Wirkart einer Peristaltikpumpe quetschbar, womit bei Bewegung der Roller 73, die im Flüssigkeitskanal befindliche Flüssigkeit oder Luft, förderbar ist. Zur Pumpeinrichtung siehe auch Fig. 8a bis Fig. 8d.

Ein erfindungsgemäß ausgebildeter Überdrucksensor 37 und/oder 37c welcher in seiner Funktion gegenüber dem Atmosphärendruck lediglich Überdrücke feststellen muss, kann in der hier gezeigten Ausführungsform beispielsweise mit einer in der Außenschale 41 der Kassette 1 zugänglichen Membranen-Fläche 37c einer Druckkammer in einem Flüssigkeitskanal der Kassette 1 ausgebildet sein, auf welche Membran-Fläche 37c bei eingebrachter, geklemmter Kassette 1 eine Sensitivitäts-Fläche eines Kraftsensors 85 im Gerät 99 flächig zur Anlage kommt. Mittels der flexiblen Membrane 37c wird die, durch den Druck der Flüssigkeit in der Kassette 1 auf die Membrane ausgeübte Kraft auf die Sensitivitäts-Fläche des Kraftsensors 85 im Gerät 99 übertragen. Der Wert dieser Kraft, welche positiv auf die Sensitivitäts-Fläche drückt und welche vom Kraftsensor 85 erfasst wird, ist dabei proportional zum Druck im Flüssigkeitskanal. Der beschriebene Kraftsensor 85 kann auch als Drucksensor ausgeführt sein. Fig. 10 illustriert eine beispielhafte erfindungsgemäße Ausführungsform.

**Fig. 7b** zeigt eine beispielhafte Ausführungsform einer erfindungsgemäßen Kassette 1, welche in ein erfindungsgemäßes Gerät 99 eingelegt ist, in einer Schittdarstellung durch die Kassette 1. Im Schnitt der Kassette 1 zu sehen sind speziell beispielhafte Flüssigkeitskanäle 98 und Pressfit 45 bzw. Schnapp-Verbinder 45 mittels welchen die Kassette 1 zusammengefügt ist. Im hinteren Bereich ist ein erfindungsgemäßer Kraftsensor 11b gezeigt, welcher für eine hier nicht gezeigte, längere Kassettenvariante im Gerät 99 vorgesehen ist.

Gezeigt ist auch eine Ausführungsform einer kassettenseitigen Codierung 70a und einer zugehörigen geräteseitigen Code-Auswertung 71a - in diesem Beispiel in Form von Schaltelementen, welche entsprechend einer Formgebung der Kassette 1 betätigt werden - Beispielsweise um unterschiedliche Ausführungsformen von Kassetten erkennen zu können. Weiters ist auch eine Beispielhafte Ausführungsform eines automatischen Einzugs für die Kassette 1 in das Gerät 99 gezeigt - in diesem Beispiel mit einer Kassettenseitigen Verzahnung und einem geräteseitigen Zahnrad, welches beim Einlegen der Kassette 1 in diese Verzahnung eingreift und mit welchem die Kassette automatisch in ihre Endlage einziehbar bwz. nach Gebrauch auswerfbar ist.

**Fig. 7c** zeigt eine beispielhafte Ausführungsform einer erfindungsgemäßen Kassette 1 vor dem Einlegen in ein Gerät 99, von welchem nur ein Teil eines Kassettenschachts dargestellt ist. Speziell gezeigt sind die erfindungsgemäßen Kraftsensoren 11 und 11b im Gerät, welche mit dem Drucksensor 10 der Kassette 1, welcher sich auf der nicht sichtbaren Rückseite der Kassette 1 befindet, zusammenwirken wird, wenn die Kassette eingelegt ist. Um das gegenüber der Außenhülle der Kassette 1 hervorragen Kupplungselement 49 aufnehmen zu können ist in Einlegerichtung jeweils hin zum Kraftsensor 11,11a eine Nut im Kassettenschacht ausgebildet. Das Einlegen erfolgt durch ein einschieben der Kassette 1 in den Kassettenschacht in Einlegerichtung 91.

**Fig. 7d** zeigt die eingelegte Kassette 1 in Ihrer Endstellung im Kassettenschacht, in welcher erfindungsgemäß der kassettenseitige Drucksensor 10 mit dem geräteseitigen Kraftsensor 11 gekoppelt ist, und auch die anderen geräteseitigen Sensoren und Aktoren bezüglich der Kassette in einer Lage sind, in welcher diese mit ihren jeweiligen Pendants in der Kassette 1 zweckgemäß zusammenwirken können.

**Fig. 8a** zeigt eine beispielhafte Ausführungsform einer erfindungsgemäßen Kassette 1 in welcher die erfindungsgemäße kassettenseitige Pumpeinrichtung 36,52 in Form einer Peristaltikpumpe dargestellt ist. Fig. 8b zeigt selbiges nochmals in einer Schnittdarstellung durch die Kassette 1.

Gezeigt sind eine erste Pumpeinrichtung 36 und eine davon getrennte, zweite Pumpeinrichtung 52, beispielsweise eine für die Infusion und eine für die Aspiration. Diese sind mit den Weichbereichen 44b der Außenschale 42 gebildet, welche nach Außen Aufgewölbte Quetschkanäle 94 ausbilden. Durch den Eingriff von Rollern 73 (also den gezeigten Rollen 73c und/oder 73d) werden diese Quetschkanäle 94a,94b,94c,94d lokal abdichtend auf den Kernteil 43 gedrückt. Durch Bewegung der Roller 73, kann damit ein Volumen in den Quetschkanälen 94 gefördert werden.

Ein weiterer, hier gezeigter erfindungsgemäßer Teilaspekt zur Verringerung von oben genannten Ripple-Effekte liegt in einer zweifachen Ausführung mit zumindest zwei benachbarten Quetschbereichen 94c und 94d. Diese sind derart ausgebildet, dass durch die Quetschpumpwirkung auftretende Ripple der benachbarten Quetschkanäle 94c und 94d sich in Summe möglichst ausgleichen und damit der Gesamt-Ripple verringert wird. Dies kann beispielsweise durch gegeneinander versetzte Quetschrollen 73c und 73d bewirkt werden, mit welchen eine Phasenverschiebung der Ripple von den beiden Quetschkanälen 94c und 94d bewirkt wird. Dabei sind die beiden Ein- sowie die beiden Auslässe der Quetschkanälen 94c und 94d jeweils miteinander verbunden. Insbesondere kann eine Phasenverschiebung der Ripple-Verläufe um ca. 180 Grad bei zwei Kanälen, eine vorteilhafte Reduktion bewirken. Bei einer anderen Anzahl von Quetschkanälen 94 ist die Phasenverschiebung entsprechend anders zu wählen. Alternativ oder zusätzlich kann dabei auch die Lage und/oder Formgebung der Ein- und/oder Auslässe der beiden Quetschbereiche 94 unterschiedlich gestaltet werden, um den besagten Phasenversatz und/oder eine weitere Ripple-Reduktion zu bewirken. Somit kann bei entsprechender Auslegung eine Ripple-Reduktion gegenüber einem einzelnen Quetschbereich erzielt wird.

Bei der hier gezeigten, zumindest annährend konzentrischen Anordnung von zwei zusammengeschalteten Quetschkanälen 94c und 94d, kann dabei zudem berücksichtigt werden, dass mit den unterschiedlichen Bogenradien auch unterschiedliche Bogenlängen auftreten. Zur Ripple-Kompensation können daher erfindungsgemäß die Flüssigkeitskanal-Querschnittsdimensionen, Querschnittsformen und/oder Querschnittsverläufe des äußeren 94c gegenüber dem innerer 94d Bogen derart unterschiedlich ausgebildet sein, dass das pro Bewegungseinheit der Rollen 73 geförderte Volumen stets zumindest annähernd gleich bleibt - respektive die Volumina sich stets möglichst ausgleichen und in Summe einen möglichst geringen Ripple des geförderten Gesamtvolumens ergeben. Beispielsweise also etwa derart, dass über eine ganze Umdrehung des die Rollen 73 tragenden Rollenkopfes hinweg die äußere sowie die innere Quetschbahn 94c und 94d zumindest annähernd jeweils dasselbe Volumen fördern. Durch allenfalls unterschiedliche Durchmesser der eingreifenden Rollen 73c und 73d bedingte, Unterschiedliche Quetschradien können bei diesen Überlegungen ebenfalls berücksichtigt werden.

**Fig. 8c****.** zeigt die Kassette 1 mit im Eingriff befindlichen, beispielhaften Rollenköpfen 72. Der Übersichtlichkeit halber ist nur der untere drehbaren Rollenkopf 72b, an welchem die Rollen 73b angebracht sind, in der Figur angedeutet - bei der oberen Pumpeinrichtung sind nur die Rollen 73a nicht jedoch der zugehörige Rollenkopf 72a dargestellt. Im hier gezeigten Beispiel greifen ein erster Rollenkopf 72a und ein zweiter Rollenkopf 72b der Pumpen auf derselben Seite der Kassette 1 ein, in einer anderen Ausführungsform kann erster und zweiter Rollenkopf 72a und 72b aber auch auf gegenüberliegenden Seiten der Kassette 1 eingreifen. Insbesondere, aber nicht nötigerweise, können dabei die Drehachsen der Rollenköpfe gegenüberliegend angeordnet sein und eine zumindest annähernd gemeinsame Drehachse ausbilden, was Vorteile bezüglich der Kraftverteilung auf die Kassette 1 bringen kann.

Im Beispiel der hier gezeigten erfindungsgemäßen Ausführungsform beschrieben die beiden Pumpen 36 und 52 zumindest annähernd jeweils einen Halbkreis auf der Kassette 1. Dieses ergibt auf der beispielhaft gezeigten Kassette 1 eine vorteilhafte Ausnutzung der Platzverhältnisse, speziell bei einfacher Handhabbarkeit und Herstellbarkeit. Eine ebenfalls ausführbare, konzentrische Anordnung der beiden Pumpen 36 und 52 wäre auch möglich, aber vergleichsweise konstruktiv aufwändiger. Alternativ kann der wulstförmige Quetschbereich 94 der Pumpe 36 und/oder 52 in einer anderen Ausführungsform auch mit einer anderen, insbesondere geringeren Bogenlänge ausgebildet sein, wobei jedoch stets eine entsprechende Anordnung und Anzahl der Rollen 73a bzw. 73b gewählt werden muss, sodass ein hinreichendes Pumpverhalten sichergestellt werden kann und in jedem Quetschbereich 94 stets zumindest eine Rolle 73a im Eingriff ist.

Um ein vorteilhaftes Abrollverhalten zu erzielen, sind erfindungsgemäß vorzugsweise, wie im gezeigten Beispiel dargestellt, die Rollen 73a und 73b nicht zylindrisch, sondern als Kegelstümpfe ausgebildet. Die Ausformung und Anordnung erfolgt dabei vorzugsweise derart, dass der Umfang der Rolle 73a bzw. 73b an jeder Stelle jeweils proportional dem Umfang ist, welcher dem von der Rolle an dieser Stelle beschriebenen Kreisbahn um das Rotationszentrum 75a,75b des Rollenkopfes 72b entspricht.

Insbesondere kann dabei erfindungsgemäß die Querschnittsfläche des dabei ausgebildeten Quetschbereichs 94 über dessen Länge variieren. Wie in diesem Beispiel einer erfindungsgemäßen Ausführungsform gezeigt, kann etwa eine Verjüngung des Querschnitts hin zum Auslass der Pumpe 36,52 und/oder eine Vergrößerung oder Verkleinerung des ungequetschten Querschnitts des Quetschbereichs 94 beim Einlass der Pumpe 36,52 ausgebildet sein. Dabei kann insbesondere mit einer Optimierung dieser Quetsch-Querschnittsveränderung eine Minimierung von Druck- und/oder Fördermengen-Schwankungen (auch als Ripple bezeichnet) während des Förderns erzielt werden. Ein vorteilhafter Querschnittsverlauf entlang des Quetschbereichs 94 kann beispielsweise auch mittels Simulation und/oder Versuchsreihen ermittelt werden.

Wie in **Fig. 8d** illustriert, kann dies beispielsweise erfindungsgemäß erzielt werden, indem die Rollenachsen 74 gegenüber deren Abrollebenen auf den Quetschkanälen 94 schräg angeordnet sind, und sich vorzugsweise im Drehzentrum 75 des die Rollen 73 tragenden Rollenkopfes 72 mit dieser Abrollebene schneiden. Die kegelstumpfförmigen Rollen 73 rollen damit optimal auf dem, die Quetschkanäle 94 bildenden Weichkunststoffbereich 44b der Außenschale 42 ab, und quetschen diesen gegen den Kernteil 43. Alternativ können aber allenfalls auch andere Geometrien genutzt werden.

**Fig. 9a** und **Fig. 9b** zeigt eine beispielhafte Ausführungsform eines erfindungsgemäßen Ventils 35,39,51,51b und/oder 57 in einer erfindungsgemäßen Kassette 1. Dabei wird mit einem geräteseitigen 99 Stößel 80a bzw. 80b auf einen, das Ventil 35,39,51,51b und/oder 57 ausbildenden, mit Weichkunststoff 44a ausgeführter Funktionsbereich der Kassette 1 eingegriffen. Mit einem erfindungsgemäßen Ventil wird ein Teilbereich des Flüssigkeitskanals der Kassette 1 in seinem Querschnitt beeinflusst. Insbesondere wird der Querschnittsbereich wie links in Fig. 9a mit 81a dargestellt, bei gehobenem Stößel 80a entweder freigegeben, oder wie rechts in Fig. 9b mit 81b dargestellt bei angedrücktem Stößel 80b verschlossen, indem ein Teilbereich des Weichkunststoffs 44a durch den Stößel 80b auf das Kernteil 43 gedrückt wird - und somit der Querschnitt des Flüssigkeitskanals an dieser Stelle verschlossen wird. Der bewegliche Stößel 80a bzw. 80b ist dabei Teil des Geräts 99. Das Ventil 35,39,51,51b und/oder 57 befindet sich an der Außenschale 41 der Kassette 1, und ein Bereich mit welchem der Flüssigkeitskanal 98b verschlossen werden kann, kann aus dem Weichkunststoff 44a der 2K-Spritzguss-Außenschale 41 ausgebildet sein, und dieser Bereich kann beim Verschließen insbesondere mit Kernteil 43 zusammenwirken.

**Fig. 10** zeigt eine beispielhafte Ausführungsform eines erfindungsgemäßen Überdrucksensors 37 und/oder 37c in einer erfindungsgemäßen Kassette 1. Bei der gezeigten Ausführungsform bildet ein Bereich des Weichkunststoffs 44a der 2K-Spritzguss-Außenschale 41 eine Membranenfläche 87 aus, welche auf deren Innenseite in hydrostatischer Verbindung mit einem Flüssigkeitskanal 98b der Kassette 1 steht, vorzugsweise von diesem durchflossen wird. Nach Einlegen der Kassette 1 in das Gerät 99 wird ein geräteseitiger Kraftsensor 85 oder Drucksensor 85 mit dessen Sensitivitätsfläche 86 von außen an der Membranenfläche 87 zur Anlage gebracht. Anhand der somit bestimmbaren - vom Druck im Flüssigkeitskanal 98b auf die Membranenfläche 87 und über diese auf die Sensitivitätsfläche 86 ausgeübte - Kraftwirkung, ist der Druck im Flüssigkeitskanal 98b erfassbar.

**Fig. 11** zeigt eine beispielhafte Ausführungsform eines erfindungsgemäßen Luftblasensensors 34 in einer erfindungsgemäßen Kassette 1. Dieser wirkt optisch, indem eine Lichtquelle 95 (z.B. eine LED) Licht in den Flüssigkeitskanal emittiert und ein Lichtsensor 96 (z.B. eine Photodiode) empfangenes Licht auswertet. Im gezeigten Beispiel kommt hierbei geräteseitig 99 ein lichtleitendes Kunststoffteil 97 zum Einsatz, welches mit dem kassettenseitigen Luftblasensensors 34 zusammenwirkt. In einer bevorzugten Ausführungsform kann ein kassettenseitiger 1 Luftblasensensors 34 mit einer optisch transparenten Hartkunststoff der Außenschale 41 ausgebildet sein, welche ein - beispielsweise poliertes - Fenster hin zu einem Bereich des Flüssigkeitskanals 98 ausbildet. Alternativ kann auch ein transparentes Einlegeteil oder eine zusätzliche transparente Komponente beim Mehr-Komponenten-Spitzgussverfahren angewandt werden.

## Patentansprüche

1. Ophthalmologische Wechselkassette (1), ausgebildet zur Verwendung mit einer ophthalmologischen Apparatur (99) - insbesondere eine mit Patientenflüssigkeit in Kontakt tretende austauschbare ophthalmologische Kassette (1) für ein ophthalmo-chirurgisches Gerät (99) - mit:
▪ zumindest einem Hartbereich (41,42,43) als Gehäuse der Wechselkassette (1), insbesondere aus einem Hartkunststoff,
▪ zumindest einem Weichbereich (44a,44b), insbesondere aus einem Weichkunststoff,
∘ wobei Hartbereich (41,42,43) und Weichbereich (44a,44b) interne Flüssigkeitskanäle der Wechselkassette (1) ausbilden, und
∘ wobei der Weichbereich (44a,44b) eine kassettenseitige Druckerfassungseinrichtung (10) zur Bestimmung eines Flüssigkeitsdruckes in zumindest einem der Flüssigkeitskanäle ausbildet,
**dadurch gekennzeichnet, dass**
die Druckerfassungseinrichtung (10) in der Wechselkassette (1) ausgebildet ist mit:
▪ einer Druckkammer (47), welche mit einem der Flüssigkeitskanäle in Verbindung steht,
▪ welche Druckkammer (47) nach Außen hin eine flexible Membrane (48) aufweist, und
▪ welche flexible Membrane (48) ein Kupplungselement (49) umlaufend einfasst,
▪ wobei das Kupplungselement (49) mit einer zumindest teilweise umlaufenden Ausnehmung (46) im Kupplungselement (49), welche im wesentlichen parallel zur Membrane (48) und zu einer Einschubrichtung (91) der Wechselkassette (1) in die ophthalmologische Apparatur (99) ausgerichtet ist, derart ausgebildet ist, dass bei einem substantiell parallel zur Membrane (49) erfolgenden Einschieben der Wechselkassette (1) in die ophthalmologische Apparatur (99) ein, zu einer Gabel ausgeformter Ausleger (11) einer Kraftmesseinrichtung (14) der ophthalmologischen Apparatur (99) substantiell parallel zur Membrane (49) in die Ausnehmung (46) derart eingreift, dass das Kupplungselement (49) zumindest zweiseitig vom gabelförmigen Ausleger (11) gefasst wird und mit der Kraftmesseinrichtung (14) eine in substanziell normaler Richtung zur Membrane (48) auftretende Kraftwirkung (92) auf die Membrane (48) in positiver sowie in negativer Richtung erfassbar ist.

2. Wechselkassette (1) nach Anspruch 1, **dadurch gekennzeichnet, dass**
die Ausnehmung (46) über mehr als 180° des Kupplungselements (49) ausgebildet ist, insbesondere wobei sich die Ausnehmung (46) über drei Seiten des Kupplungselements (49) erstreckt, insbesondere wobei die Ausnehmung (46) derart ausgebildet ist um den gabelförmigen Ausleger (11) über einen Winkelbereich von mehr als 180° aufzunehmen.

3. Wechselkassette (1) nach zumindest einem der Ansprüche 1 bis 2, **dadurch gekennzeichnet, dass**
die Ausnehmung (46) zum direkten Eingriff des Auslegers (11), als einer mit Dehnmessstreifen versehene Platte mit gabelförmiger Ausnehmung, in die Ausnehmung (46) des Kupplungselements (49) ausgebildet und angeordnet ist.

4. Wechselkassette (1) nach zumindest einem der Ansprüche 1 bis 3, **dadurch gekennzeichnet, dass**
das Kupplungselement (49) mit seiner Ausnehmung (11) derart ausgebildet ist, dass mit zwei Zinken der Gabel (11) in die Ausnehmung (46) des Kupplungselements (49) derart eingreifbar ist, dass das Kupplungselement (49) an der Ausnehmung (46) zumindest zweiseitig, insbesondere dreiseitig, von diesen Zinken gefasst wird.

5. Wechselkassette (1) nach zumindest einem der Ansprüche 1 bis 4, **dadurch gekennzeichnet, dass**
die Membrane (48) und das Kupplungselement (49) einstückig aus demselben Material ausgebildet sind und die Ausnehmung (46) mit einer Anschrägung (45) ausgebildet ist.

6. Wechselkassette (1) nach zumindest einem der Ansprüche 1 bis 5, **dadurch gekennzeichnet, dass**
der Weichbereich (44a,44b) Dichtungen (93) der Drucckammer (47) und/oder der Flüssigkeitskanäle (94) sowie weitere Funktionselemente der Wechselkassette (1) ausbildet, welche Funktionselemente zumindest:
• einen Quetschpumpbereich (36,52) für Flüssigkeit in den Flüssigkeitskanälen für eine Volumenförderung in Form einer Peristaltikpumpe,
• einen Ventileinrichtungsbereich (35,39,57,51) zur Veränderung eines Durchflussquerschnitts in zumindest einem der Flüssigkeitskanäle,
• einen Überdrucksensorbereich (37) zur Bestimmung eines Flüssigkeitsdruckes in zumindest einem der Flüssigkeitskanäle, und/oder
• einen passiven, flexiblen Ripple-Kompensationsbereich (59) zum Ausgleich von Druck- und/oder Volumen-Schwankungen einer Flüssigkeitsförderung durch einen elastischen Flüssigkeitskanalbereich
ausbilden.

7. Wechselkassette (1) nach zumindest einem der Ansprüche 1 bis 6, **dadurch gekennzeichnet, dass** die Wechselkassette (1) zudem weiters zumindest mit einer Auswahl aus,
∘ einem Flaschenanschluss (33) zum Anschluss eines Behälters (30) zur Bereitstellung von Infusionsflüssigkeit,
∘ einem Infusionsanschluss (38) zur Zuführung der Infusionsflüssigkeit zum ophthalmo-chirurgischen Operationsinstrument
∘ einem Aspirationsanschluss (50) zur Verbindung von zumindest einem der Flüssigkeitskanäle mit einem ophthalmo-chirurgischen Operationsinstrument, und
∘ einem Abfall-Anschluss (55) zur Abfuhr von Flüssigkeit in ein Abfallgebinde (56), und/oder
∘ einem Infusions-Ventil (35)
ausgebildet ist und wobei ein mit dem Aspirationsanschluss (50) verbundener Flüssigkeitskanal mit der Druckerfassungseinrichtung (10) in Verbindung steht.

8. Wechselkassette (1) nach zumindest einem der Ansprüche 1 bis 7, **dadurch gekennzeichnet, dass** der Hartbereich aus einem Hartkunststoff - insbesondere aus einem Thermoplast wie z.B. einem Polypropylen (PP) oder Polyethylen (PE) - und der Weichbereich aus einem Weichkunststoff - insbesondere aus einem thermoplastischen Elastomer (TPE) - ausgebildet ist, und wobei Hartkunststoff und Weichkunststoff als ein einstückiges Mehrkomponenten-Spritzgussteil ausgebildet sind.

9. Wechselkassette (1) nach einem der Ansprüche 1 bis 8, **dadurch gekennzeichnet, dass**
die Wechselkassette (1) mit:
einer ersten Außenschale (41) und einer zweiten Außenschale (42), jeweils aus dem 2K-Spritzguss des Hart- und Weichkunststoffs, welche das Gehäuse der Wechselkassette (1) bilden, sowie
einem Kernteil (43) aus Hartkunststoff, aufgebaut ist, wobei das Kernteil (43) zwischen den Außenschalen (41,42) angeordnet ist, und die erste Außenschale (41) und zweite Außenschale (42) und/oder das Kernteil (43) zu einer fertigen Wechselkassette (1) miteinander verschnappt oder kaltverpresst sind - insbesondere wobei die fertiggestellte Wechselkassette (1) dabei klebstofffrei und schweißnahtfrei ist - und das Kupplungselement 46 der Druckerfassungseinrichtung (10) eine der Außenschalen (41,42) zumindest teilweise überragt.

10. Gerät (99) in Form einer ophtalmologischen Apparatur mit welcher eine Saugspülung während eines ophthalmologischen Eingriffs durchführbar ist, welches zur Aufnahme einer Ausführungsform einer Wechselkassette (1) nach einem der Ansprüche 1 bis 9 mit einem schlitzförmigen Aufnahmeschacht ausgebildet ist, welcher einen gabelförmigen Ausleger (11) einer Kraftmesseinrichtung (14) aufweist, welcher Ausleger (11) derart ausgebildet und angeordnet ist um, bei einem linearen Einschieben einer Wechselkassette (1) in den Aufnahmeschacht, in die zumindest teilweise umlaufende Ausnehmung (46) des Kupplungselements (49) der Druckerfassungseinrichtung (10) der Wechselkassette (1) zumindest zweiseitig einzugreifen,
- insbesondere wobei das Gerät (99) im Aufnahmeschacht weitere Aktoren und Sensoren aufweist welche derart ausgebildet und angeordnet sind, um mit Funktionselementen der Wechselkassette (1) zu interagieren.

11. Gerät (99) nach Anspruch 10, **dadurch gekennzeichnet, dass**
der Ausleger der Kraftmesseinrichtung (11) parallel zu einer Richtung des linearen Einschiebens und mit seiner Gabelöffnung in Richtung einer Öffnung des Aufnahmeschachts ausgerichtet ist, und derart ausgebildet ist, dass das Kupplungselement (49) der Wechselkassette (1) beim linearen Einschieben der Wechselkassette (1) in den Aufnahmeschacht selbsttätig eine Wirkverbindung mit dem Ausleger (11) eingeht, mit welcher Wirkverbindung eine orthogonal zur Richtung des Einschiebens auftretende Kraftwirkung (92) auf das Kupplungselement (49) in positiver und negativer Richtung auf die Kraftmesseinrichtung (14) übertragbar ist, speziell indem das gabelförmige Endes des Auslegers (11) zumindest zweiseitig in die Ausnehmung (48) des Kupplungselements (49) eingreift.

12. Ophthalmologisches System mit welchem eine Saugspülung während eines ophthalmologischen Eingriffs durchführbar ist, mit
einem Gerät (99) nach einem der Ansprüche 10 oder 11 sowie mit einer in den Aufnahmeschacht des Geräts (99) in einer substantiell linearen Einschubrichtung (91) einschiebbaren, ebensolchen Wechselkassette (1) nach einem der Ansprüche 1 bis 9,
wobei der gabelförmigen Ausleger (11) des Geräts (99) in Einschubrichtung (91) in die Ausnehmung (46) des Kupplungselements (49) der Wechselkassette (1) zumindest zweiseitig umschließend eingreift.

13. Kopplungsverfahren für eine Wechselkassette (1) nach zumindest einem der Ansprüche 1 bis 9 mit einem ophthalmologischen Gerät (99) nach zumindest einem der Ansprüche 10 bis 11, bei welchem ein Koppeln einer kassettenseitigen Druckmesseinrichtung (10) mit einem linearen Einschieben eines, mit einer zumindest teilweise umlaufenden Ausnehmung (46) ausgebildeten, Kupplungselements (49) der kassettenseitigen Druckmesseinrichtung (10),
in eine gabelförmige Ausbuchtung eines Auslegers der geräteseitigen Kraftmesseinrichtung (11) erfolgt, insbesondere wobei das lineare Einschieben substantiell parallel zur Ausnehmung (46) des Kupplungselements (49) der kassettenseitigen Druckmesseinrichtung (10) sowie zur gabelförmige Ausbuchtung des Ausleger der geräteseitigen Kraftmesseinrichtung (11) erfolgt.

14. Verfahren zur Aspirationsdruckmessung mit einem System nach Anspruch 12, bei welchem die Druckmessmembrane (48) der Wechselkassette (1) und der Ausleger (11) des Geräts (99) substantiell parallel zueinander ausgerichtet sind und der Ausleger (11) in einer dazu ebenfalls substantiell parallelen Einschubrichtung (91) linear in die Ausnehmung (46) des Kupplungselements (49) der Druckmessmembrane (48) eingeschoben ist, wobei mit der Kraftmesseinrichtung (14) eine in substanziell normaler Richtung zur Druckmessmembrane (48) auftretende Kraftwirkung (92) auf die Druckmessmembrane (48) in positiver sowie in negativer Richtung erfasst, welche proportional zum Aspirationsdruck im Flüssigkeitskanal ist.

15. Verfahren zur Herstellung einer ophthalmologischen Wechselkassette (1) nach zumindest einem der Ansprüche 1 bis 9, mit
einem Mehrkomponenten-Spritzgießen eines Hartkunststoffs und eines Weichkunststoffs zu einer jeweils einstückigen ersten und zweiten Außenschale (41,42), welche Außenschalen (41,42) in dem Hartkunststoff mechanisch flexibel ausgebildete Funktionselemente aus dem Weichkunststoff aufweisen, welche Funktionselemente zumindest eine kassettenseitige Druckerfassungseinrichtung (10) ausbilden, wobei die Druckerfassungseinrichtung (10) mit einer Membrane (48), welche ein zumindest teilweise umlaufend mit einer Ausnehmung (46) ausgeformtes Kupplungselement (49) ausbildet, und
einem unlösbaren Verschnappen oder Verpressen der ersten und zweiten Außenschalen (41,42), wobei der Weichkunststoff Dichtungen von Flüssigkeitskanälen im inneren der Wechselkassette (1) ausbildet,
wobei zwischen erster und zweiter Außenschale (41,42) ein Kernteil (43) aus Hartkunststoff eingelegt wird und wobei die resultierende Wechselkassette (1) dabei klebstofffrei und schweißnahtfrei ausgebildet ist.

## Claims

1. A ophthalmologic replacement cassette (1) configured for a utilization with an ophthalmological apparatus (99) - in particular an interchangeable ophthalmological cassette (1) coming into contact with patient liquid for an ophthalmic surgical device (99) - with:
▪ at least one hard region (41, 42, 43) as housing of the replacement cassette (1), in particular made of a hard plastic,
▪ at least one soft region (44a, 44b), in particular made of a soft plastic,
∘ wherein the hard region (41, 42, 43) and the soft region (44a, 44b) form inner liquid channels of the replacement cassette (1), and
∘ wherein the soft region (44a, 44b) forms a cassette-side pressure detection arrangement (10) for determining a liquid pressure in at least one of the liquid channels,
**characterized in that**
the pressure detection arrangement (10) in the replacement cassette (1) is configured with:
▪ a pressure chamber (47), which is connected to one of the liquid channels,
▪ which pressure chamber (47) has a flexible membrane (48) outwardly, and
▪ which flexible membrane (48) peripherally surrounds a coupling element (49),
▪ wherein the coupling element (49) is configured with an at least partially circumferential recess (46) in the coupling element (49), which is oriented substantially parallel to the membrane (48) and to an insertion direction (91) of the replacement cassette (1) into the ophthalmological apparatus (99), which is configured in such a way that, as the replacement cassette (1) is inserted substantially parallel to the membrane (48) into the ophthalmological apparatus (99), an arm (11), shaped into a fork, of a force measuring arrangement (14) of the ophthalmological apparatus (99) engages substantially parallel to the membrane (48) into the recess (46),
in such a way that the coupling element (49) is surrounded at least on two sides by the fork-shaped arm (11) and a force effect (92) on the membrane (48) occurring in a direction substantially normal in relation to the membrane (48) is detectable by the force measuring arrangement (14) both in a positive and in a negative direction.

2. The replacement cassette (1) according to claim 1, **characterized in that**
the recess (46) is formed over more than 180° of the coupling element (49), in particular wherein the recess (46) extends over three sides of the coupling element (49), in particular wherein the recess (46) is formed in such a way as to receive the fork-shaped arm (11) over an angular range of more than 180°.

3. The replacement cassette (1) according to at least one of claims 1 to 2, **characterized in that** the recess (46) is configured and arranged for direct engagement of the arm (11), in form of a plate configured with a strain gauge and a fork-shaped recess, in the recess (46) of the coupling element (49) .

4. The replacement cassette (1) according to at least one of claims 1 to 3, **characterized in that** the coupling element (49) is configured with its recess (11) in such a way that two prongs of the fork (11) are engageable in the recess (46) of the coupling element (49) in such a way that the recess (46) of the coupling element is engaged by these prongs on at least two sides, in particular on three sides.

5. The replacement cassette (1) according to at least one of claims 1 to 4, **characterized in that** the membrane (48) and the coupling element (49) are formed in one piece of a single material and the recess (46) is configured with a chamfer (45).

6. The replacement cassette (1) according to at least one of claims 1 to 5, **characterized in that** the soft region (44a, 44b) form seals (93) of the pressure chamber (47) and/or the liquid channels (94) as well as further functional elements of the replacement cassette (1), which functional elements form
• a squeeze pump region (36, 52) for liquid in the liquid channels, for a volume delivery in the form of a peristaltic pump,
• a valve arrangement region (35, 39, 57, 51) for varying a flow cross-section in at least one of the liquid channels,
• a positive pressure sensor region (37) for determining a liquid pressure in at least one of the liquid channels, and/or
• a passive, flexible ripple compensation region (59) for balancing pressure and/or volume fluctuations of a liquid delivery by means of an elastic liquid channel region.

7. The replacement cassette (1) according to at least one of claims 1 to 6, **characterized in that** the replacement cassette (1) is furthermore formed with a selection from
∘ a flange connection (33) for the connection of a container (30) for providing infusion liquid,
∘ an infusion connection (38) for feeding the infusion liquid to the ophthalmic surgical instrument,
∘ an aspiration connection (50) for connection of at least one of the liquid channels to an ophthalmic surgical instrument, and
∘ a waste connection (55) for discharging liquid into a waste container (56), and/or
∘ an infusion valve (35),
and wherein a liquid channel connected to the aspiration connection (50) is connected to the pressure detection arrangement (10).

8. The replacement cassette (1) according to at least one of claims 1 to 7, **characterized in that** the hard region is made of a hard plastic - in particular made of a thermoplastic, such as a polypropylene (PP) or polyethylene (PE) - and the soft region is made of a soft plastic - in particular made of a thermoplastic elastomer (TPE) - and wherein hard plastic and soft plastic are formed as an integral multi-component injection-molded part.

9. The replacement cassette (1) according to any one of claims 1 to 8, **characterized in that** the replacement cassette (1) is configured with:
a first outer shell (41) and a second outer shell (42), each formed from two-component injection molding of the hard and soft plastic, which form the housing of the replacement cassette (1), and
a core part (43) made of hard plastic, wherein the core part (43) is arranged between the outer shells (41, 42), and the first outer shell (41) and second outer shell (42) and/or the core part (43) are snapped or cold-pressed with one another to form a finished replacement cassette (1) - in particular wherein the finished replacement cassette (1) is free from adhesive and free from weld seams - and the coupling element (46) of the pressure detection arrangement (10) protrudes beyond one of the outer shells (41, 42) at least in part.

10. A device (99) in the form of an ophthalmological apparatus configured for performing a suction-flushing during an ophthalmological intervention, which device is configured to receive an embodiment of a replacement cassette (1) according to any one of claims 1 to 9 with a slot-shaped receiving shaft, which is configured with a fork-shaped arm (11) of a force measuring arrangement (14), which arm (11) is designed and arranged in such a way as to engage at least on two sides into the at least partially circumferential recess (46) of the coupling element (49) of the pressure detection arrangement (10) of the replacement cassette (1), as the replacement cassette (1) is inserted linearly into the receiving shaft - in particular wherein the device (99) is configured with further actuators and sensors in the receiving shaft which are configured and arranged in such a way as to interact with functional elements of the replacement cassette (1).

11. The device (99) according to claim 10, **characterized in that** the arm of the force measuring arrangement (11) is oriented parallel to a direction of the linear insertion and with its fork opening in direction of an opening of the receiving shaft, and is configured in such a way that the coupling element (49) of the replacement cassette (1), as the replacement cassette (1) is inserted linearly into the receiving shaft, enters automatically into an operative connection with the arm (11), by means of which operative connection a force effect (92) occurring orthogonal to the direction of the insertion is transmittable to the coupling element (49) both in a positive and negative direction, especially by the fork-shaped end of the arm (11) being engaged at least in two sides in the recess (46) of the coupling element (49).

12. Ophthalmologic system configured for a suction-flushing during an ophthalmological intervention, with a device (99) according to any one of the claims 10 or 11 and with a replacement cassette (1) according to at least one of claims 1 to 9, that is insertable into a the slot-shaped receiving shaft of the device (99) in a substantially linear insertion direction (91), wherein the fork-shaped arm (11) of the device (99) is engaging in the insertion direction (91) into the recess (46) of the coupling element (49) of the replacement cassette (1) in an enclosing manner at least on two sides.

13. A coupling method for coupling a replacement cassette (1) according to at least one of claims 1 to 9 with an ophthalmological device (99) according to at least one of claims 10 to 11, with a coupling of a cassette-side pressure measuring arrangement (10) is done with a linear inserting of a coupling element (49), that is configured with an at least partially circumferential recess (46), of the cassette-side pressure measuring arrangement (10) into a fork-shaped indentation of an arm of a device-side force measuring arrangement (11), in particular wherein the linear inserting is done substantially parallel to the recess (46) of the coupling element of the cassette-side side pressure measuring arrangement (10) and also to the fork-shaped indentation of the arm of the device-side force measuring arrangement (11).

14. A method for aspiration pressure measuring in a system according to claim 12, in which the membrane (48) of the replacement cassette (1) and the arm (11) of the device (99) are oriented substantially parallel to each other, and wherein the arm (11) is linearly inserted into the recess (46) of the coupling element (49) of the membrane (48), in an insertion direction (91) that is also substantially parallel thereto, wherein the force measuring arrangement (14) is detecting a force effect (92) on the membrane (48) occurring in a direction substantially normal in relation to the membrane (48) both in a positive and negative direction, which force effect (92) is proportional to the aspiration pressure in a fluid channel.

15. Method of manufacturing an ophthalmologic replacement cassette (1) according to any one of claims 1 to 9, with a multi-component injection molding of a hard plastic and a soft plastic to form a first and second outer shell (41, 42), in each case in one piece, which outer shells (41, 42) has mechanically flexible functional elements of the soft plastic in the hard plastic, which functional elements form at least one cassette-side pressure detection arrangement (10), wherein the pressure detection arrangement (10) is configured with an at least partially circumferential recess(46) of a coupling element (49) of a membrane (48), and
a non-releasable snapping or pressing of the first and second outer shells (41, 42), wherein the soft plastic forms seals of liquid channels inside the replacement cassette (1), wherein a core part (43) made of hard plastic is inserted between the first and second outer shell (41, 42) and wherein the resulting replacement cassette (1) is free of adhesive and weld seams.

## Revendications

1. Cassette ophtalmologique interchangeable (1) conçue pour être utilisée avec un appareil ophtalmologique (99) - en particulier une cassette ophtalmologique interchangeable (1) qui entre en contact avec un liquide du patient pour un appareil ophtalmo-chirurgical (99) - comprenant :
▪ au moins une zone dure (41, 42, 43) servant de boîtier de la cassette interchangeable (1), en particulier constituée d'une matière plastique dure,
▪ au moins une zone souple (44a, 44b), en particulier constituée d'une matière plastique souple,
∘ la zone dure (41, 42, 43) et la zone souple (44a, 44b) formant des canaux de liquide internes de la cassette interchangeable (1), et
∘ la zone souple (44a, 44b) formant un moyen de détection de pression (10) côté cassette pour déterminer une pression de liquide dans au moins l'un des canaux de liquide,
**caractérisée en ce que**
le moyen de détection de pression (10) est formé dans la cassette interchangeable (1) avec :
▪ une chambre de pression (47) qui est reliée à l'un des canaux de liquide,
▪ laquelle chambre de pression (47) présente une membrane flexible (48) vers l'extérieur, et
▪ laquelle membrane flexible (48) entoure périphériquement un élément d'accouplement (49),
▪ l'élément d'accouplement (49) avec un évidement (46) au moins partiellement périphérique dans l'élément d'accouplement (49), lequel est orienté sensiblement parallèlement à la membrane (48) et à une direction d'insertion (91) de la cassette interchangeable (1) dans l'appareil ophtalmologique (99), étant conçu de telle sorte que, lorsque la cassette interchangeable (1) est insérée dans l'appareil ophtalmologique (99) sensiblement parallèlement à la membrane (49), un bras en porte-à-faux (11) en forme de fourche d'un moyen de mesure de force (14) de l'appareil ophtalmologique (99) s'engage dans l'évidement (46) sensiblement parallèlement à la membrane (49) de telle sorte que l'élément d'accouplement (49) est saisi au moins sur deux côtés par le bras en porte-à-faux (11) en forme de fourche et qu'un effet dynamique (92) agissant sur la membrane (48) dans une direction sensiblement normale à la membrane (48) peut être détecté dans un sens positif ainsi que dans un sens négatif avec le moyen de mesure de force (14).

2. Cassette interchangeable (1) selon la revendication 1, **caractérisée en ce que** l'évidement (46) est formé sur plus de 180° de l'élément d'accouplement (49), l'évidement (46) s'étendant en particulier sur trois côtés de l'élément d'accouplement (49), l'évidement (46) étant en particulier formé de manière à recevoir le bras en porte-à-faux (11) en forme de fourche sur une plage angulaire de plus de 180°.

3. Cassette interchangeable (1) selon au moins l'une des revendications 1 à 2, **caractérisée en ce que** l'évidement (46) est conçu et disposé pour l'engagement direct du bras en porte-à-faux (11), sous la forme d'une plaque munie de jauges de contrainte et présentant un évidement en forme de fourche, dans l'évidement (46) de l'élément d'accouplement (49) .

4. Cassette interchangeable (1) selon au moins l'une des revendications 1 à 3, **caractérisée en ce que** l'élément d'accouplement (49) avec son évidement (11) est conçu de telle sorte que deux dents de la fourche (11) peuvent être engagées dans l'évidement (46) de l'élément d'accouplement (49) de telle sorte que l'élément d'accouplement (49) soit saisi au niveau de l'évidement (46) au moins sur deux côtés, en particulier sur trois côtés, par ces dents.

5. Cassette interchangeable (1) selon au moins l'une des revendications 1 à 4, **caractérisée en ce que** la membrane (48) et l'élément d'accouplement (49) sont formés d'une seule pièce à partir du même matériau et l'évidement (46) est formé avec un biseau (45).

6. Cassette interchangeable (1) selon au moins l'une des revendications 1 à 5, **caractérisée en ce que** la zone souple (44a, 44b) forme des joints (93) de la chambre de pression (47) et/ou des canaux de liquide (94) ainsi que d'autres éléments fonctionnels de la cassette interchangeable (1), lesquels éléments fonctionnels forment au moins :
• une zone de pompe à écrasement (36, 52) pour le liquide dans les canaux de liquide pour un transport de volume sous la forme d'une pompe péristaltique,
• une zone de moyen de soupape (35, 39, 57, 51) pour modifier une section d'écoulement dans au moins un des canaux de liquide,
• une zone de capteur de surpression (37) pour déterminer une pression de liquide dans au moins un des canaux de liquide et/ou
• une zone de compensation d'ondulation passive flexible (59) pour compenser les fluctuations de pression et/ou de volume d'un transport de liquide à travers une zone de canal de liquide élastique.

7. Cassette interchangeable (1) selon au moins l'une des revendications 1 à 6, **caractérisée en ce que** la cassette interchangeable (1) est formée en outre au moins avec une sélection
∘ d'un raccord de bouteille (33) pour raccorder un récipient (30) servant à fournir un liquide de perfusion,
∘ d'un raccord de perfusion (38) pour amener le liquide de perfusion à l'instrument d'opération ophtalmo-chirurgical,
∘ d'un raccord d'aspiration (50) pour raccorder au moins un des canaux de liquide à un instrument d'opération ophtalmo-chirurgical, et
∘ d'un raccord de déchets (55) pour l'évacuation de liquide dans un conteneur de déchets (56), et/ou
∘ d'une soupape de perfusion (35)
et dans laquelle un canal de liquide relié au raccord d'aspiration (50) est relié au moyen de détection de pression (10).

8. Cassette interchangeable (1) selon au moins l'une des revendications 1 à 7, **caractérisée en ce que** la zone dure est formée d'une matière plastique dure - en particulier d'une matière thermoplastique telle que, par exemple, un polypropylène (PP) ou un polyéthylène (PE) - et la zone souple est formée d'une matière plastique souple - en particulier d'un élastomère thermoplastique (TPE) - et dans laquelle la matière plastique dure et la matière plastique souple sont réalisées sous la forme d'une seule pièce moulée par injection à plusieurs composants.

9. Cassette interchangeable (1) selon l'une des revendications 1 à 8, **caractérisée en ce que** la cassette interchangeable (1) est constituée :
d'une première coque extérieure (41) et d'une deuxième coque extérieure (42), chacune étant issue du moulage par injection à deux composants des matières plastiques dure et souple, qui forment le boîtier de la cassette interchangeable (1), ainsi que
d'une partie centrale (43) en matière plastique dure, la partie centrale (43) étant disposée entre les coques extérieures (41, 42), et la première coque extérieure (41) et la deuxième coque extérieure (42) et/ou la partie centrale (43) étant assemblées par encliquetage ou par pressage à froid pour former une cassette interchangeable finie (1) - en particulier, la cassette interchangeable finie (1) étant exempte de colle et de soudures - et l'élément d'accouplement 46 du moyen de détection de pression (10) faisant saillie au moins partiellement de l'une des coques extérieures (41, 42).

10. Appareil (99) sous la forme d'un appareil ophtalmologique avec lequel un rinçage par aspiration peut être effectué lors d'une intervention ophtalmologique, qui est conçu avec un puits de réception en forme de fente pour recevoir un mode de réalisation d'une cassette interchangeable (1) selon l'une des revendications 1 à 9, lequel présente un bras en porte-à-faux (11) en forme de fourche d'un moyen de mesure de force (14), lequel bras en porte-à-faux (11) est conçu et disposé pour, lors d'une insertion linéaire d'une cassette interchangeable (1) dans le puits de réception, s'engager au moins sur deux côtés dans l'évidement (46) au moins partiellement périphérique de l'élément d'accouplement (49) du moyen de détection de pression (10) de la cassette interchangeable (1),
l'appareil (99) présentant en particulier dans le puits de réception d'autres actionneurs et capteurs qui sont conçus et disposés de manière à interagir avec des éléments fonctionnels de la cassette interchangeable (1).

11. Appareil (99) selon la revendication 10, **caractérisé en ce que** le bras en porte-à-faux du moyen de mesure de force (11) est orienté parallèlement à une direction d'insertion linéaire et avec son ouverture de fourche dans la direction d'une ouverture du puits de réception, et est conçu de telle sorte que l'élément d'accouplement (49) de la cassette interchangeable (1) entre automatiquement en liaison active avec le bras en porte-à-faux (11) lors de l'insertion linéaire de la cassette interchangeable (1) dans le puits de réception, avec laquelle liaison active un effet dynamique (92) agissant orthogonalement à la direction d'insertion sur l'élément d'accouplement (49) dans les sens positif et négatif peut être transmis au moyen de mesure de force (14), en particulier l'extrémité en forme de fourche du bras en porte-à-faux (11) s'engageant au moins sur deux côtés dans l'évidement (48) de l'élément d'accouplement (49).

12. Système ophtalmologique avec lequel un rinçage par aspiration peut être effectuée lors d'une intervention ophtalmologique, comprenant un appareil (99) selon l'une des revendications 10 ou 11 ainsi qu'une cassette interchangeable (1) selon l'une des revendications 1 à 9 pouvant être insérée dans le puits de réception de l'appareil (99) dans une direction d'insertion (91) sensiblement linéaire,
dans lequel le bras en porte-à-faux (11) en forme de fourche de l'appareil (99) s'engage, dans la direction d'insertion, (91) dans l'évidement (46) de l'élément d'accouplement (49) de la cassette interchangeable (1) de manière à l'entourer au moins sur deux côtés.

13. Procédé d'accouplement d'une cassette interchangeable (1) selon au moins l'une des revendications 1 à 9 avec un appareil ophtalmologique (99) selon au moins l'une des revendications 10 à 11, dans lequel un accouplement d'un moyen de mesure de pression (10) côté cassette est réalisé par insertion linéaire d'un élément d'accouplement (49) du moyen de mesure de pression (10) côté cassette, lequel élément d'accouplement est formé avec un évidement (46) au moins partiellement périphérique, dans un renflement en forme de fourche d'un bras en porte-à-faux du moyen de mesure de force (11) côté appareil,
en particulier dans lequel l'insertion linéaire a lieu sensiblement parallèlement à l'évidement (46) de l'élément d'accouplement (49) du moyen de mesure de pression (10) côté cassette et au renflement en forme de fourche du bras en porte-à-faux du moyen de mesure de force (11) côté appareil.

14. Procédé de mesure de pression d'aspiration avec un système selon la revendication 12, dans lequel la membrane de mesure de pression (48) de la cassette interchangeable (1) et le bras en porte-à-faux (11) de l'appareil (99) sont orientés sensiblement parallèlement l'une à l'autre et le bras en porte-à-faux est inséré linéairement dans l'évidement (46) de l'élément d'accouplement (49) de la membrane de mesure de pression (48) dans une direction d'insertion (91) également sensiblement parallèle à ceux-ci, dans lequel le moyen de mesure de force (14) détecte un effet dynamique (92) agissant sur la membrane de mesure de pression (48) dans une direction sensiblement normale à la membrane de mesure de pression (48) dans un sens positif ainsi que dans un sens négatif, qui est proportionnel à la pression d'aspiration dans le canal de liquide.

15. Procédé de fabrication d'une cassette ophtalmologique interchangeable (1) selon au moins l'une des revendications 1 à 9, avec
un moulage par injection à plusieurs composants d'une matière plastique dure et d'une matière plastique souple pour former respectivement une première et une deuxième coque extérieure (41, 42) d'une seule pièce, lesquelles coques extérieures (41, 42) présentent des éléments fonctionnels en matière plastique souple qui sont mécaniquement flexibles dans la matière plastique dure, lesquels éléments fonctionnels forment au moins un moyen de détection de pression (10) côté cassette, le moyen de détection de pression (10) étant pourvu d'une membrane (48) qui forme un élément d'accouplement (49) formé avec un évidement (46) au moins partiellement périphérique, et avec
un encliquetage ou un pressage non détachable des première et deuxième coques extérieures (41, 42),
la matière plastique souple formant des joints de canaux de liquide à l'intérieur de la cassette interchangeable (1),
une partie centrale (43) en matière plastique dure étant insérée entre les première et deuxième coques extérieures (41, 42), et
la cassette interchangeable (1) résultante étant formée sans colle et sans soudures.
